# EUROPEAN PATENT APPLICATION

(11) **EP 2 415 483 A1**
(43) Date of publication of application: **08.02.2012**
(21) Application number: 11174173.2
(22) Date of filing: 25.07.2006
(51) Int. Cl.: A61K 45/00, A61K 39/395, A61P 19/02, A61P 31/00

(54) **Single dose use of cd20-specific binding molecules**

(30) Priority: 25.07.2005 US 702498 P; 27.07.2005 US 702875 P
(62) Divisional of application: 06800341.7
(71) Applicant: Emergent Product Development Seattle, LLC, Seattle, Washington 98121 (US)
(72) Inventor: Burge, Daniel, Jonathan, Sammamish, WA 98075 (US)
(74) Representative: Walker, Ross Thomson

(57) **Abstract**

The present invention provides materials and methods for treatment of diseases involving aberrant B-cell activity using a single dose of CD20-specific binding molecule.

SEQUENCE LISTING

<110>  Burge, Daniel J.

<120>  Single Dose Use of CD20-Specific Binding Molecules

<130>  31126/41325PCT

<160>  2

<170>  PatentIn version 3.3

<210>  1
<211>  1518
<212>  DNA
<213>  artificial sequence

<220>
<223>  TRU-015 polynucleotide

<400>  1
aagcttgccg ccatggattt tcaagtgcag attttcagct tcctgctaat cagtgcttca      60

gtcataatgt ccagaggaca aattgttctc tcccagtctc cagcaatcct gtctgcatct     120

ccaggggaga aggtcacaat gacttgcagg gccagctcaa gtgtaagtta catgcactgg     180

taccagcaga agccaggatc ctcccccaaa ccctggattt atgccccatc caacctggct     240

tctggagtcc ctgctcgctt cagtggcagt gggtctggga cctcttactc tctcacaatc     300

agcagagtgg aggctgaaga tgctgccact tattactgcc agcagtggag ttttaaccca     360

cccacgttcg gtgctgggac caagctggag ctgaaagatg gcggtggctc gggcggtggt     420

ggatctggag gaggtgggag ctctcaggct tatctacagc agtctggggc tgagtcggtg     480

aggcctgggg cctcagtgaa gatgtcctgc aaggcttctg gctacacatt taccagttac     540

aatatgcact gggtaaagca gacacctaga cagggcctgg aatggattgg agctatttat     600

ccaggaaatg gtgatacttc ctacaatcag aagttcaagg gcaaggccac actgactgta     660

gacaaatcct ccagcacagc ctacatgcag ctcagcagcc tgacatctga agactctgcg     720

gtctatttct gtgcaagagt ggtgtactat agtaactctt actggtactt cgatgtctgg     780

ggcacaggga ccacggtcac cgtctctgat caggagccca aatcttgtga caaaactcac     840

acatctccac cgtgctcagc acctgaactc ctgggtggac cgtcagtctt cctcttcccc     900

ccaaaaccca aggacaccct catgatctcc cggacccctg aggtcacatg cgtggtggtg     960

gacgtgagcc acgaagaccc tgaggtcaag ttcaactggt acgtggacgg cgtggaggtg    1020

cataatgcca agacaaagcc gcgggaggag cagtacaaca gcacgtaccg tgtggtcagc    1080

gtcctcaccg tcctgcacca ggactggctg aatggcaagg agtacaagtg caaggtctcc    1140

aacaaagccc tcccagcccc catcgagaaa accatctcca aagccaaagg gcagccccga    1200

gaaccacagg tgtacaccct gcccccatcc cgggatgagc tgaccaagaa ccaggtcagc    1260

ctgacctgcc tggtcaaagg cttctatcca agcgacatcg ccgtggagtg ggagagcaat    1320

gggcagccgg agaacaacta caagaccacg cctcccgtgc tggactccga cggctccttc   1380

ttcctctaca gcaagctcac cgtggacaag agcaggtggc agcaggggaa cgtcttctca   1440

tgctccgtga tgcatgaggc tctgcacaac cactacacgc agaagagcct ctccctgtct   1500

ccgggtaaat gatctaga                                                 1518

<210>  2
<211>  499
<212>  PRT
<213>  artificial sequence

<220>
<223>  TRU-015 polypeptide

<400>  2

Met Asp Phe Gln Val Gln Ile Phe Ser Phe Leu Leu Ile Ser Ala Ser
1               5                   10                  15

Val Ile Met Ser Arg Gly Gln Ile Val Leu Ser Gln Ser Pro Ala Ile
            20                  25                  30

Leu Ser Ala Ser Pro Gly Glu Lys Val Thr Met Thr Cys Arg Ala Ser
        35                  40                  45

Ser Ser Val Ser Tyr Met His Trp Tyr Gln Gln Lys Pro Gly Ser Ser
    50                  55                  60

Pro Lys Pro Trp Ile Tyr Ala Pro Ser Asn Leu Ala Ser Gly Val Pro
65                  70                  75                  80

Ala Arg Phe Ser Gly Ser Gly Ser Gly Thr Ser Tyr Ser Leu Thr Ile
                85                  90                  95

Ser Arg Val Glu Ala Glu Asp Ala Ala Thr Tyr Tyr Cys Gln Gln Trp
            100                 105                 110

Ser Phe Asn Pro Pro Thr Phe Gly Ala Gly Thr Lys Leu Glu Leu Lys
        115                 120                 125

Asp Gly Gly Gly Ser Gly Gly Gly Gly Ser Gly Gly Gly Gly Ser Ser
    130                 135                 140

Gln Ala Tyr Leu Gln Gln Ser Gly Ala Glu Ser Val Arg Pro Gly Ala
145                 150                 155                 160

Ser Val Lys Met Ser Cys Lys Ala Ser Gly Tyr Thr Phe Thr Ser Tyr
                165                 170                 175

Asn Met His Trp Val Lys Gln Thr Pro Arg Gln Gly Leu Glu Trp Ile

180                 185                 190

Gly Ala Ile Tyr Pro Gly Asn Gly Asp Thr Ser Tyr Asn Gln Lys Phe
        195                 200                 205

Lys Gly Lys Ala Thr Leu Thr Val Asp Lys Ser Ser Ser Thr Ala Tyr
    210                 215                 220

Met Gln Leu Ser Ser Leu Thr Ser Glu Asp Ser Ala Val Tyr Phe Cys
225                 230                 235                 240

Ala Arg Val Val Tyr Tyr Ser Asn Ser Tyr Trp Tyr Phe Asp Val Trp
                245                 250                 255

Gly Thr Gly Thr Thr Val Thr Val Ser Asp Gln Glu Pro Lys Ser Cys
            260                 265                 270

Asp Lys Thr His Thr Ser Pro Pro Cys Ser Ala Pro Glu Leu Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser His
305                 310                 315                 320

Glu Asp Pro Glu Val Lys Phe Asn Trp Tyr Val Asp Gly Val Glu Val
                325                 330                 335

His Asn Ala Lys Thr Lys Pro Arg Glu Glu Gln Tyr Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Ala Leu Pro Ala Pro Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Arg Asp Glu Leu Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro

        435                     440                     445

Val Leu Asp Ser Asp Gly Ser Phe Phe Leu Tyr Ser Lys Leu Thr Val
    450                     455                     460

Asp Lys Ser Arg Trp Gln Gln Gly Asn Val Phe Ser Cys Ser Val Met
465                     470                     475                 480

His Glu Ala Leu His Asn His Tyr Thr Gln Lys Ser Leu Ser Leu Ser
                485                     490                     495

Pro Gly Lys

## Description

This application claims the priority benefit of U.S. Provisional Patent Application Nos. 60/702,498, filed July 25, 2005, and 60/702,875, filed July 27, 2006, each of which is herein incorporated by reference.

### FIELD OF THE INVENTION

The present invention provides materials and methods for treatment of diseases involving aberrant B-cell activity using a single dose of a CD20-specific binding molecule.

### BACKGROUND OF THE INVENTION

In its usual role, the human immune system protects the body from damage from foreign substances and pathogens. One way in which the immune system protects the body is by production of specialized cells called B lymphocytes or B-cells. B-cells produce antibodies that bind to, and in some cases mediate destruction of, a foreign substance or pathogen.

In some instances though, the human immune system, and specifically the B lymphocytes of the human immune system, go awry and disease results. There are numerous cancers that involve uncontrolled proliferation of B-cells. There are also numerous autoimmune diseases that involve B-celf production of antibodies that, instead of binding to foreign substances and pathogens, bind to parts of the body. In addition, there are numerous autoimmune and inflammatory diseases that involve B-cells in their pathology, for example, through inappropriate B-cell antigen presentation to T-cells or through other pathways involving B-cells. For example, autoimmune-prone mice deficient in B-cells do not develop autoimmune kidney disease, vasculitis or autoantibodies. (Shlomchik et al., J Exp. Med. 1994,180:1295-306). Interestingly, these same autoimmune-prone mice which possess B-cells but are deficient in immunoglobulin production, do develop autoimmune diseases when induced experimentally (Chan et al., J Exp. Med. 1999, 189:1639-48), indicating that B-cells play an integral role in development of autoimmune disease.

B-cells can be identified by molecules on their cell surface CD20 was the first human B-cell lineage-specific surface molecule identified by a monoclonal antibody. It is a non-glycosylated, hydrophobic 35 kDa B-cell transmembrane phosphoprotein that has both its amino and carboxy ends situated inside the cell. Einfeld et al., EMBO J. 1988, 7:711-17. CD20 is expressed by all normal mature B-cells, but is not expressed by precursor B-cells or plasma cells. Natural ligands for CD20 have not been identified, and the function of CD20 in B-cell biology is still incompletely understood.

Anti-CD20 monoclonal antibodies affect the viability and growth of B-cells. (Clark et al., Proc. Natl. Acad. Sci. USA 1936, 83:4494-98). Extensive cross-linking of CD20 can induce apoptosis in B lymphoma cell lines (Shan et al., Blood 1998, 91:1644-52), and cross-linking of CD20 on the cell surface has been reported to increase the magnitude and enhance the kinetics of signal transduction, for example, as detected by measuring tyrosine phosphorylation of cellular substrates. (Deans et al., J. Immunol. 1993, 146:846-53). Therefore, in addition to cellular depletion by complement and ADCC mechanisms, Fc-receptor binding by CD20 monoclonal antibodies *in vivo* may promote apoptosis of malignant B-celis by CD20 cross-linking, consistent with the theory that effectiveness of CD20 therapy of human lymphoma in a SCID mouse model may be dependent upon Fc-receptor binding by the CD20 monoclonal antibody (Funakoshi et al., J. Immunotherapy 1996, 19:93-101*).* The presence of multiple membrane spanning domains in the CD20 polypeptide (Einfeld et al., EMBO J. 1988, 7:711-17; Stamenkovic et al., J. Exp. Med. 1988, 167:1975-80; Tedder et al., J. Immunol. 1988.141:4388-4394), prevent CD20 internalization after antibody binding, and this was recognized as an important feature for therapy of B-cell malignancies when a murine CD20 monoclonal antibody, 1F5, was injected into patients with B-cell lymphoma, resulting in significant depletion of malignant cells and partial clinical responses (Press et al., Blood 1987, 69:584-91).

Because normal mature B-cells also express CD20, normal B-cells are depleted by anti-CD20 antibody therapy (Reff et al., Blood 1994, 83:435-445). After treatment is completed, however, normal B-cells can be regenerated from CD20 negative B-cell precursors, therefore, patients treated with anti-CD20 therapy do not experience significant immunosuppression.

GD20 is expressed by malignant cells of B-cell origin, including B-cell lymphoma and chronic lymphocytic leukemia (CLL). CD20 is not expressed by malignancies of pre-B-cells, such as acute lymphoblastic leukemia, cD20 is therefore a good target for therapy of B-cell lymphoma, CLL, and other diseases in which B-cells are involved in the disease etiology. Other B-cell disorders include autoimmune diseases in which autoantibodies are produced during the differentiation of B-cells into plasma cells.

Various groups have investigated the use of anti-CD20 antibodies to treat B-cell related diseases. One treatment consists of anti-CD20 antibodies prepared in the form of radionuclides for treating B-cell lymphoma (e.g., ¹³¹I-labeled anti-CD20 antibody), as well as a ⁸⁹Sr-labeled form for the palliation of bone pain caused by prostate and breast cancer metastases (Endo, Gan To Kagaku Ryoho 1999, 26, 744-748).

Patents and patent publications concerning CD20 antibodies include U.S. Pat. Nos. 5,776,456, 5,736,137, 6,399,061, and 5,843,439, as well as US Patent Application Nos. US 2002/0197255A1 and US 2003/0021781A1 (Anderson et al.); U.S. Pat. No. 6,455,04381 and WO 00/09160 (Grillo-Lopez, A.); WO 00/27428 (Grillo-Lopez and White); WO 00/27433 (Grillo-Lope; and Leonard); WO 00/44788 (Brastawsky et al.); WO 01/10462 (Rastetter, W.); WO 01/10461 (Rastetter and White); WO 01/10460 (White and Grillo-Lopez); US Application No. US2002/0006404 and WO 02/04021 (Hanna and Hariharan); US Application No. US2002/0012665 A1 and WO 01/74388 (Hanna, N.); US Application No. US2002/0009444A1, and WO 01/80S84 (Grillo-Lopez, A.); WO 01/97858 (White, C.); US Application No. US2002/0128488A1 and WO 02/34790 (Reff, M.);WO 02/060955 (Braslawsky et al.);WO 02/096948 (Braslawsky et al.);WO 02/079255 (Reff and Davies); U.S. Pat. No. 6,171,586B1, and WO 98/56418 (Lam et al.); WO 98/58964 (Raju, S.); WO 99/22764 (Raju, S.);WO 99/51642, U.S. Pat. No. 6,194,551B1, U.S. Pat. No. 6,242,19561, U.S. Pat. No. 6.528,62481 and U.S. Pat. No. 6,538,124 (Idusogie et al.); WO 00/42072 (Presta, L.); WO 00/67796 (Curd et al.); WO 01/03734 (Grillo-Lopez et al.); US Application No. US 2002/0004587A1 and WO 01/77342 (Miller and Presta); US Application No. US2002/0197256 (Grewal. I.); U.S. Pat. Nos. 6,090,365B1. 6,287,537B1, 6,015,542, 5,843,398, and 5,596,721, (Kaminski et al.); U.S. Pat. Nos. 5,500,362, 5,677,180, 5,721,108, and 6,120,767 (Robinson et al.); U.S. Pat No. 6,410,391 B1 (Raubitschek et al.); U.S. Pat. No, 6,224,866B1 and WO 00/20864 (Barbera-Guillem, E.); WO 01/13945 (Barbera-Guillem, E.); WO 00/67795 (Goldenberg); WO 00/74718 (Goldenberg and Hansen); WO 00/76542 (Golay et al.);WO 01/72333 (Wolin and Rosenblatt); U.S. Pat. No. 6,368,596B1 (Ghetie et al.); US Application No. US2002/0041847A1, (Goldenberg, D.); US Application no. US2003/0026801A1 (Weiner and Hartmann); WO 02/102312 (Engleman, E.), See, also, U.S. Pat. No. 5,849,898 and EP Application No. 330,191 (Seed et al.); U.S. Pat. No. 4,861,579 and EP332,865A2 (Meyer and Weiss); and WO 95/03770 (Bhat et al.), each of which is expressly incorporated herein by reference,

A chimeric monoclonal antibody specific for CD20, consisting of heavy and light chain variable regions of mouse origin fused to human IgG1 heavy chain and human kappa light chain constant regions, reportedly retained binding to CD20 and the ability to mediate ADCC and fix complement (Liu et al., J. Immunol. 1987, 139:3521-26). Yet another chimeric anti-CD20 antibody was made from IDEC hybridoma C2B8 and was named rituximab. The mechanism of anti-tumor activity of rituximab, discussed above, is thought to be a combination of several activities, including antibody-dependent cell-mediated cytotoxicity (ADCC), complement fixation, and triggering of signals that promote apoptosis in malignant B-colls. ADCC is a cell-mediated reaction in which nonspecific cytotoxic cells that express Fc receptors (FcRs) (e.g. Natural Killer (NK) cells, neutrophils, and macrophages) recognize bound antibody on a target cell and subsequently cause lysis of the target cell. Complement fixation, or complement-dependent cytotoxity (CDC) is the ability of a molecule to lyse a target in the presence of complement. The complement activation pathway is initiated by the binding of the first component of the complement system (C1q) to a molecule (e.g. an antibody) complexed with a cognate antigen. The large size of rituximab prevents optimal diffusion of the molecule into lymphoid tissues that contain malignant B-cells, thereby limiting these anti-tumor activities.

Rituximab, typically administered in 4 doses as 4 weekly infusions of antibody (1 dose/week x 4), is currently used to treat low-grade or follicular B-cell non-Hodgkin's lymphoma (McLaughlin et al., Oncology 1998, 12: 1763-1777; Leget et al., Curr. Opin. Oncol. 1998,10: 548-551) and in relapsed stage III/IV follicular lymphoma (White et al., Pharm. Sci. Technol. Today 1999, 2: 95-101). Other disorders treatable with Rituximab include follicular centre cell lymphoma (FCC), mantle cell lymphoma (MCL), diffuse large cell lymphoma (DLCL), and small lymphocytic lymphoma (SLL) (Nguyen et al., Eur J Haematol. 1999, 62:76-82)). Rituximab administered in weekly infusions is also used to treat CLL (Lin et al., Sem Onco/. 2003, 30:483-92).

Anti-CD20 antibodies have also been used extensively to treat patients suffering from autoimmune diseases associated with B-cell production of autoantibodies. For example, rituximab has demonstrated significant clinical benefit in depleting CD20+ B-rells in patients with multiple autoimmune/inflammatory diseases including RA (Edwards, N Engl J Med. 2004, 350:2546-8; Cambridge et al., Arthritis Rheum. 2003, 48:2146-54). RA patients received continued doses of methotrexate (MTX) and a 2 dose course of rituximab infusion (Edwards et al., *supra*). These patients showed improved American College of Rheumatology (ACR) responses compared to control groups.

In a trial for the treatment of systemic lupus erythematosus (SLE) (Leandro et al., Arthritis Rheum. 2002,46:2673-7), patients were administered two infusions of high dose rituximab, and demonstrated B-cell depletion and improved disease state, In a second study of B-cell depletion in SLE (Looney et al., Arthritis Rheum. 2004, 50:2580-9), patients were given a single infusion of 100 mg/m2 (low dose), a single infusion of 375 mg/m2 (intermediate dose), or as 4 infusions (1 week apart) of 375 mg/m2 (high dose). These patients demonstrated B-cell depletion and improved disease scores, but the treatment did not alter the level of autoantibody. Trials of Rituximab have also been carried out in Walden strom's macroglobulinemia (Treon et al., Immunother. 2001, 24:272-9), where patients showed increased hematocrit (HCT) and platelet (PLT) counts after 4 infusions of rituximab.

Recent reports of rituximab treatment in patients suffering from multiple scleorosis, an autoimmune disease affecting the central nervous system, indicate that a course of rituximab treatment depletes peripheral B-cells but has little effect on B-cells in cerebrospinal fluid (Monson et al., Arch Neural. 2005, 62:258-64),

Additional publications concerning the use of rituximab includes: Stashi et al. "Rituximab chimeric anti-CD20 monoclonal antibody treatment for adults with chronic idiopathic thrombocytopenic purpura" Blood 2001, 98:952-957; Matthews, R. "Medical Heretics" New Scientist (7 Apr., 2001); Leandro et al. "Clinical outcome in 22 patients with rheumatoid arthritis treated with B lymphocyte depletion" Ann Rheum Dis 2002, 61:833-888; Leandro et al. "Lymphocyte depletion in rheumatoid arthritis: early evidence for safety, efficacy and dose response. Arthritis and Rheumatism 2001, 44:S370; Leandro et al. "An open study of B lymphocyte depletion in systemic lupus erythematosus", Arthritis Rheum. 2002,46:2673-2677; Edwards et al., "Sustained improvement in rheumatoid arthritis following a protocol designed to deplete B lymphocytes" Rheumatology 2001, 40:205-211; Edwards et al. "B-lymphocyte depletion therapy in rheumatoid arthritis and other autoimmune disorders" Biochem. Soc. Trans. 2002,30:824-828; Edwards et al. "Efficacy and safety of Rituximab, a B-cell targeted chimeric monoclonal antibody: A randomized, placebo controlled trial in patients with rheumatoid arthritis. Arthritis Rheum. 2002,46: S197; Levine et al., "IgM antibody-related polyneuropathies: B-cell depletion chemotherapy using Rituximab" Neurology 1999, 52:1701-1704; DeVita et al. "Efficacy of selective B-cell blockade in the treatment of rheumatoid arthritis" Arthritis Rheum. 2002, 46:2029-2033: Hidashida et al. "Treatment of DMARD-Refractory rheumatoid arthritis with rituximab." Presented at the Annual Scientific Meeting of the American College of Rheumatology; October 24-29; New Orleans, La. 2002; Tuscano, J. "Successful treatment of infliximab-refractory rheumatoid arthritis with rituximab" Presented at the Annual Scientific Meeting of the American College of Rheumatology; October 24-29; New Orleans, La. 2002.

Problems associated with rituximab therapy remain. For example, the majority of cancer patients treated with rituximab relapse, generally within about 6-12 months, and fatal infusion reactions within 24 hours of rituximab infusion have been reported. These fatal reactions followed an infusion reaction complex that included hypoxia, pulmonary infiltrates, acute respiratory distress syndrome, myocardial Infarction, ventricular fibrillation or cardiogenic shock. Acute renal failure requiring dialysis with instances of fatal outcome has also been reported in the setting of tumor lysis syndrome following treatment with rituximab, as have severe mucocutaneous reactions, some with fatal outcome. Additionally, high doses of rituximab are required for intravenous injection because the molecule is large, approximately 150 kDa, and, as noted above, diffusion into the lymphoid tissues where many tumor cells reside is limited. A further disadvantage of rituximab treatment is that multiple doses of rituximab are typically given to patients receiving therapy, usually a high dose every week for four weeks (Maloney et al., Blood 1997, 90:2188-2195), but a dose response study in humans given a single dose of rituximab antibody resulted in modest B-cell depletion in subjects receiving high doses of antibody (Maloney et al., Blood 1994, 84:2457-2466).

Monoclonal antibody technology and genetic engineering methods have led to rapid development of immunoglobulin molecules for diagnosis and treatment of human diseases. Protein engineering has been applied to improve the affinity of an antibody for its cognate antigen, to diminish problems related to immunogenicity, and to alter an antibody's effector functions. The domain structure of immunoglobulins is amenable to engineering, in that the antigen binding domains and the domains conferring effector functions may be exchanged between immunogtobulin classes and subclasses. Immunoglobulin structure and function are reviewed, for example, in Harlow et al., Eds., Antibodies: A Laboratory Manual, Chapter 14, Cold Spring Harbor Laboratory, Cold Spring Harbor (1988), An extensive introduction as well as detailed information about all aspects of recombinant antibody technology can be found in the textbook "Recombinant Antibodies" (John Wiley & Sons, NY, 1999). A comprehensive collection of detailed antibody engineering lab protocols can be found in R. Kontermann and S. Dübel (eds.), "The Antibody Engineering Lab Manual" (Springer Verlag, Heidelberg/New York, 2000).

Recently, smaller immunoglobulin molecules have been constructed to overcome problems associated with whole immunoglobulin therapy. Single chain Fv (scFv) comprise an antibody heavy chain variable domain joined via a short linker peptide to an antibody light chain variable domain (Huston et al., Proc. Natl. Acad. Sci. USA, 1988, 85: 5879-83). In addition to variable regions, each of the antibody chains have one or more constant regions. Light chains have a single constant region domain. Thus, light chains have one variable region and one constant region. Heavy chains have several constant region domains. The heavy chains in IgG, IgA, and IgD antibodies have three constant region domains, which are designated CH1, CH2, and CH3, and the heavy chains in IgM and IgE antibodies have four constant region domains, CH1, CH2, CH3 and CH4. Thus, heavy chains have one variable region and three or four constant regions.

The heavy chains of immunoglobulins can also be divided into three functional regions: the Fd region (a fragment comprising V_{H} and CH1, i.e., the two N-terminal domains of the heavy chain), the hinge region, and the Fc region (the "fragment crystallizable" region, derived from constant regions and formed after pepsin digestion). The Fd region in combination with the light chain forms an Fab (the "fragment antigen-binding"). Because an antigen will react stereochemically with the antigen-binding region at the amino terminus of each Fab the IgG molecule is divalent, i.e., it can bind to two antigen molecules. The Fc contains the domains that interact with immunoglobulin receptors on cells and with the initial elements of the complement cascade. Thus, the Fc fragment is generally considered responsible for the effector functions of an immunoglobulin, such as complement fixation and binding to Fc receptors.

Because of the small size of scfv molecules, they exhibit very rapid clearance from plasma and tissues and more effective penetration into tissues than whole immunoglobulin, An anti-tumor scFv showed more rapid tumor penetration and more even distribution through the tumor mass than the corresponding chimeric antibody (Yokota et al., Cancer Res. 1992, 52:3402-08). Fusion of an scFv to another molecule, such as a toxin, takes advantage of the specific antigen-binding activity and the small size of an scFv to deliver the toxin to a target tissue. (Chaudary et al., Nature 1989, 339:394); Batra et al., Mol. Cell. Biol. 1991, 11:2200).

Despite the advantages that scfv molecules bring to serotherapy, several drawbacks to this therapeutic approach exist. While rapid clearance of scFv may reduce toxic effects in normal cells, such rapid clearance may prevent delivery of a minimum effective dose to the target tissue. Manufacturing adequate amounts of scFv for administration to patients has been challenging due to difficulties in expression and isolation of scFv that adversely affect the yield, During expression, scFv molecules lack stability and often aggregate due to pairing of variable regions from different molecules. Furthermore, production levels of scFv molecules in mammalian expression systems are low, limiting the potential for efficient manufacturing of scFv molecules for therapy (Davis et al, J Biol. Chem. 1990, 265-10410-18); Traunecker et al., EMBO J 1991, 10: 3655-59). Strategies for improving production have been explored, including addition of glycosylafion sites to the variable regions (Jost, C. R. U.S. Pat. No. 5,888,773, Jost et al, J. Biol. Chem. 1994, 69: 26267-73).

Another disadvantage to using scFv for therapy is the lack of effector function. An scfv without the cytolytic functions, ADCC and complement dependent-cytotoxicity (CDC), associated with the constant region of an immunoglobulin may be ineffective for treating disease. Even though development of scFv technology began over 12 years ago, currently no scFv products are approved for therapy.

Alternatively, it has been proposed that fusion of an scFv to another molecule, such as a toxin, could take advantage of the specific antigen-binding activity and the small size of an scFv to deliver the toxin to a target tissue. Ghaudary et al., Nature 1989,339:394; Batra et al., Mol. Cell. Biol. 1991,11:2200. Conjugation or fusion of toxins to scFvs has thus been offered as an alternative strategy to provide potent, antigen-specific molecules, but dosing with such conjugates or chimeras can be limited by excessive and/or non-specific toxicity due to the toxin moiety of such preparations. Toxic effects may include supraphysiological elevation of liver enzymes and vascular leak syndrome, and other undesired effects. In addition, immunotoxins are themselves highly immunogenic upon administration to a host, and host antibodies generated against the immunotoxin limit potential usefulness for repeated therapeutic treatments of an individual.

Other engineered fusion proteins, termed small, modular immunopharmaceutical (SMIP™) products, are described in co-owned US Patent Publications 2003/133939, 2003/0118592, and 2005/0136049, and co-owned International Patent Publications WO 02/056910, WO 2005/037989, and WO 2005/017148, which are all incorporated by reference herein. SMIP products are novel binding domain-immunoglobulin fusion proteins that feature a binding domain for a cognate structure such as an antigen, a counterreceptor or the like; a wild-type IgG1, IgA or IgE hinge region polypeptide or a mutant IgG1 hinge region polypeptide having either zero, one or two cysteine residues; and immunoglobulin CH2 and CH3 domains. SMIP products are capable of ADCC and/or CDC.

Although there has been extensive research carried out on antibody-based therapies, there remains a need in the art for improved methods to treat diseases associated with aberrant B-cell activity. The methods of the present invention described and claimed herein provide such improved methods as well as other advantages.

### SUMMARY OF THE INVENTION

The present invention relates to methods for modulating B-cell population levels in a disease associated with aberrant B-cell activity.

In one aspect, the invention provides a method of treating a subject having or suspected of having a disease associated with aberrant B-cell activity, comprising administering to a patient a single dose of a therapeutically effective amount of a CD20-specific binding molecule. In one embodiment, the CD20-specific binding molecule is a CD20-specific small, modular immunopharmaceutical (SMIP).

"Aberrant B-cell activity" refers to cell activity that deviates from the normal, proper, or expected course, For example, aberrant cell activity may include inappropriate proliferation of cells whose DNA or other cellular components have become damaged or defective. Aberrant B-cell activity may include cell proliferation whose characteristics are associated with a disease caused by, mediated by, or resulting in inappropriately high levels of cell division, inappropriately low levels of apoptosis, or both. Such diseases may be characterized, for example, by single or multiple local abnormal proliferations of cells, groups of cells or tissue(s), whether cancerous or non-cancerous, benign or malignant, described more fully below. Aberrant B-cell activity may also include aberrant antibody production, such as production of autoantibodies, or overproduction of antibodies typically desirable at normal levels. It is contemplated that aberrant B-cell activity may occur in certain subpopulations of B-cells and not in other subpopulations. Aberrant B-cell activity may also include inappropriate stimulation of T-cells, such as by inappropriate B-cell antigen presentation to T-cells or by other pathways involving B-cells.

A "single dose" of CD20-specific binding molecule refers to administration of a single continuous infusion of one dose of a CD20-specific SMIP at the outset of treatment, in contrast to multiple dose therapies which require once weekly or once bi-weekly administration of a CD20-specific, binding molecule. In one embodiment, a single continuous infusion may be a prolonged subcutaneous infusion, intravenous infusion, or the like, The single continuous infusion may be administered for a period of time, e.g., from about 15 minutes up to about 1 hour, about 2 hours, about 3 hours, about 4 hours, about 5 hours, about 6 hours, about 7 hours, about 8 hours, about 9 hours, about 10 hours, about 11, hours, about 12 hours, about 24 hours, or one or more days or weeks (e.g., a sustained release from an implanted device or gel depot). The single dose may be administered in conjunction with other therapeutics or second agents, and may be administered concurrently with, before or after administration of a second therapeutic agent as described wherein. According to the present invention, a single continuous infusion may encompass brief interruptions of the infusion as required by practical considerations.

"A subject having or suspected of having a disease associated with aberrant B-cell activity" is a subject in which a disease or a symptom of a disease may be caused by aberrant B-cell activity, may be exacerbated by aberrant B-cell activity, or may be relieved by regulation of B-cell activity. Examples of such diseases are B cell cancers (such as B-cell lymphoma, a B-cell leukemia, a B-cell myeloma), a disease characterized by autoantibody production or a disease characterized by inappropriate T-cell stimulation, such as by inappropriate B-cell antigen presentation to T-cells or by other pathways involving B-cells.

In one aspect, an individual treated by methods of the invention demonstrates an improved response to treatment with the CD20-binding molecule described herein which is better than the response to treatment with rituximab and no other CD20-binding molecule. A response which is improved over treatment with rituximab and no other CD20-binding molecule refers to a clinical response wherein treatment by the method of the invention results in a clinical response in a patient that is better than a clinical response in a patient receiving rituximab therapy, such as rituximab alone or rituximab in combination with other agents, wherein the other agents are not other CD20-binding molecules. An improved response is assessed by comparison of clinical criteria well-known in the art and described herein. Exemplary criteria include, but are not limited to, duration of B cell depletion, reduction in B cell numbers overall, reduction in B cell numbers in a biological sample, reduction in tumor size, reduction in the number of tumors existing and/or appearing after treatment, and improved overall response as assessed by patients themselves and physicians, e.g., using an International Prognostic Index. The improvement may be in one or more than one of the clinical criteria. An improved response with the method of the invention may be due to an inadequate response to previous or current treatment with rituximab, for example, because of toxicity and/or inadequate efficacy of the rituximab treatment.

In a related aspect, the individual treated by the methods of the invention is also administered rituximab. In one embodiment, rituximab may have been administered as a first line of treatment and continue when treatment with a method of the invention is begun. In another embodiment, rituximab treatment is discontinued after treatment with a method of the invention has begun.

"A subject having or suspected of having a rheumatic disease" is a subject or individual affected by a disease or disorder of articular origin or of the musculoskeletal system, affecting such areas as joints, cartilage, muscles, nerves, and tendons. It is further contemplated that the subject having or suspected of having a rheumatic disease may have previously received therapy to treat a rheumatic disease. In one embodiment, the rheumatic disease includes, but is not limited to, rheumatoid arthritis, ankylosing spondylitis, dermatomyositis, Henoch Schonlein purpura, juvenile rheumatoid arthritis, psoriatic arthritis, Raynaud's syndrome, Reiter's syndrome, sarcoidosis, spondyloarthropathies, progressive systemic sclerosis and myositis.

"A subject having or suspected of having a central nervous system autoimmune disease" or "central nervous system disorder" is a subject or individual affected by a disease or disorder affecting the central nervous system, including the brain and spinal cord, or such areas as the optic nerve. It is further contemplated that subject having or suspected a central nervous system disorder may have previously received therapy to treat a central nervous system disorder. In one embodiment, the central nervous system autoimmune disease includes, but is not limited to, multiple sclerosis, allergic encephalomyelitis, neuromyelitis optica, lupus myelitis and lupus cerebritis.

"Vasculitis" refers to a disease or disorder associated with inflammation in a blood vessel. Exemplary vasculitis disorders include, but are not limited to, Behcet's disease, central nervous system vasculitis, Churg-Strauss syndrome, cryoglobulinemia, glant cell arteritis, Henoch Schonlein purpura, hypersensitivity vasculitis/anglitis, Kawasaki disease, leucocytoclastic vasculitis, polyantitis, polyarteritis nodosa, polymyalgia, polychondritis, rheumatoid vasculitis, Takayasu's arteritis, Wegener's granulamatosis, vasculitis due to hepatitis, familiar Mediterranean fever, microscopic polyanglitis, Cogan's syndrome, Whiskott-Aldrich syndrome and thromboangiitis obliterans.

"Treatment" or "treating" refers to either a therapeutic treatment or prophylactic or preventative treatments. A therapeutic treatment may improve at least one symptom of disease in an individual receiving treatment or may delay worsening of a progressive disease in an individual, or prevent onset of additional associated diseases.

A "therapeutically effective dose" or "effective dose" of a CD20-specific binding molecule refers to that amount of the compound sufficient to result in amelioration of one or more symptoms of the disease begin treated. When applied to an individual active ingredient, administered alone, a therapeutically effective dose refers to that ingredient alone. When applied to a combination, a therapeutically elective dose refers to combined amounts of the active ingredients that result in the therapeutic effect, whether administered in combination, serially or simultaneously. The invention specifically contemplates that one or more CD20-specific binding molecules may be administered according to methods of the invention, each in an effective dose.

Methods contemplated by the invention are useful for treating diseases such as B cell cancers (for example, B-cell lymphomas, B-cell leukemias, B-cell lymphomas), diseases characterized by autoantibody production or diseases characterized by inappropriate T-cells stimulation of T-cells, such as by inappropriate B-cell antigen to T-cells or by other pathways invoking B-cells.

B-cell cancers include B-cell lymphomas [such as various forms of Hodgkin's disease, non-Hodgkins lymphoma (NHL) or central nervous system lymphomas], leukemias [such as acute lymphoblastic leukemia (ALL), chronic lymphocytic leukemia (CLL), Hairy cell leukemia and chronic myoblastic leukemia] and myelomas (such as multiple myeloma). Additional B cell cancers include small lymphocytic lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extranodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma/leukemia, B-cell proliferations of uncertain malignant potential, lymphomatoid granulomatosis, and post-transplant lymphoproliferative disorder.

Disorders characterized by autoantibody production are often considered autoimmune diseases. Autoimmune diseases include, but are not limited to: arthritis, rheumatoid arthritis, juvenile rheumatoid arthritis, osteoarthritis, polychondritis, psoriatic arthritis, psoriasis, dermatitis, polymyositis/dermatomyositis, inclusion body myositis, inflammatory myositis, toxic epidermal necrolysis, systemic scleroderma and sclerosis, CREST syndrome, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, systemic lupus erythematosus (SLE), subacute cutaneous lupus erythematosus, discoid lupus, lupus myelitis, lupus cerebritis, juvenile onset diabetes, multiple sclerosis, allergic encephalomyelitis, neuromyelitis optica, rheumatic fever, Sydenham's chorea, immune responses associated with acute and delayed hypersensitivity mediated by cytokines and T-lymphocytes, tuberculosis, sarcoidosis, granulomatosis including Wegener's granulomatosis and Churg-Strauss disease, agranulocytosis, vasculitis (including hypersensitivity vasculitis/anglitis, ANCA and rheumatoid vasculitis), aplastic anemia, Diamond Blackfan anemia, immune hemolytic anemia including autoimmune hemolytic anemia (AlHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Behcet disease, Castleman's syndrome, Goodpasture's syndrome, Lambert-Eaton Myasthehic Syndrome, Reynaud's syndrome, Sjorgen's syndrome, Stevens-Johnson syndrome, solid organ transplant rejection, graft versus host disease (GVHD), pemphigoid bullous, pemphigus, autoimmune polyendocrinopathies, seronegative spondyloarthropathies, Reiter's disease, stiff-man syndrome, glant cell arteritis, immune complex nephritis, IgA nephropathy, IgM polynouropathies or IgM mediated neuropathy, idiopathic thrombocytopenic purpura (ITP), thrombotic throbocytopenic purpura (TTP), Henoch-Schonlein purpura, autoimmune thrombocytopenia, autoimmune disease of the testis and ovary including autoimmune orchitis and oophoritis, primary hypothyroidism; autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Type I diabetes also referred to as insulin-dependentdiabetes mellitus (IDDM) and Sheehan's syndrome; autoimmune hepatitis, lymphoid interstitial pneumonitis (HIV), bronchiolitis obliterans (non-transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), polyarteritis nodosa (PAN) ankylosing spondylitis, Berger's disease (IgA nephropathy), rapidly progressive glomerulonephritis, primary biliary cirrhosis, Celiac sprue (gluten enteropathy), cryoglobulinemia, cryoglobulinemia associated with hepatitis, amyotrophic lateral sclerosis (ALS), coronary artery disease, familial Mediterranean fever, microscopic polyanglitis, Cogan's syndrome, Whiskott-Aldrich syndrome and thromboangiitis obliterans.

Rheumatoid arthritis (RA) is a chronic disease characterized by inflammation of the joints, leading to swelling, pain, and loss of function. Patients having RA for an extended period usually exhibit progressive joint destruction, deformity, disability and even premature death.

Systemic Lupus Erythematosus (SLE) is an autoimmune disease caused by recurrent injuries to blood vessels in multiple organs, including the kidney, skin, and joints. In patients with SLE, a faulty interaction between T cells and B-cells results in the production of autoantibodies that attack the cell nucleus. There is general agreement that autoantibodies are responsible for at least some aspects of SLE. It is contemplated that new therapies that deplete the B-cell lineage, allowing the immune system to reset as new B-cells are generated from precursors, would offer hope for long lasting benefit in SLE patients.

Multiple sclerosis (MS) is also an autoimmune disease. It is characterized by inflammation of the central nervous system and destruction of myelin, which insulates nerve cell fibers in the brain, spinal cord, and body. Although the cause of MS is unknown, it is widely believed that autoimmune T cells are primary contributors to the pathogenesis of the disease. However, high levels of antibodies are present in the cerebral spinal fluid of patients with MS, and some theories predict that the B-cell response leading to antibody production is important for mediating the disease.

Crohn's disease and a related disease, ulcerative colitis, are the two main disease categories that belong to a group of illnesses called inflammatory bowel disease (IBD). Crohn's disease is a chronic disorder that causes inflammation of the digestive or gastrointestinal (GI) tract. Although it can involve any area of the GI tract from the mouth to the anus, it most commonly affects the small intestine and/or colon, In ulcerative colitis, the GI involvement is limited to the colon.

Crohn's disease may be characterized by antibodies against neutrophil antigens, i.e., the "perinuclear anti-neutrophil antibody" (pANCA), and Saccharomyces cervisiae, i.e. the "anti-Saccharomyces cervisiae antibody" (ASCA). Many patients with ulcerative colitis have the pANCA antibody in their blood, but not the ASCA antibody, while many Grohn's patients exhibit ASCA antibodies, and not pANCA antibodies. One method of evaluating Crohn's disease is using the Crohn's disease Activity Index (CDAI), based on 18 predictor variables scores collected by physicians. CDAI values of 150 and below are associated with quiescent disease; values above that indicate active disease, and values above 450 are seen with extremely severe disease (Best, et al. "Development of a Crohn's disease activity index." Gastroenterology 70-439-444, 1976. However, since the original study, some researchers use a 'subjective value' of 200 to 250 was an healthy score.

Autoimmune thyroid disease results from the production of autoantibodies that either stimulate the thyroid to cause hyperthyroidism (Graves' disease) or destroy the thyroid to cause hypothyroidism (Hashimoto's thyroiditis). Stimulation of the thyroid is caused by autoantibodies that bind and activate the thyroid stimulating hormone (TSH) receptor. Destruction of the thyroid is caused by autoantibodies that react with other thyroid antigens.

Sjogren's syndrome is an autoimmune disease characterized by destruction of the body's moisture-producing glands.

Immune thrombocytopenic purpura (ITP) is caused by autoantibodies that bind to blood platelets and cause their destruction.

Myasthenia Gravis (MG) is a chronic autoimmune neuromuscular disorder characterized by autoantibodies that bind to acetylcholine receptors expressed at neuromuscular junctions leading to weakness of the voluntary muscle groups.

Psoriasis, is characterized by autoimmune inflammation in the skin and also associated with arthritis in 30% of cases.

Also contemplated is the treatment of idiopathic inflammatory myopathy (IIM), including dermatomyositis (DM) and polymyositis (PM). Inflammatory myopathies have been categorized using a number of classification schemes. Miller's classification schema (Miller, Rheum Dis Clin North Am. 1994, 20:811-826) identifies 2 idiopathic inflammatory myopathies (IIM), polymyositis (PM) and dermatomyositis (DM).

Polymyositis and dermatomyositis are chronic, debilitating inflammatory diseases that involve muscle and, in the case of DM, skin. These disorders are rare, with a reported annual incidence of approximately 5 to 10 cases per million adults and 0.6 to 3.2 cases per million children per year in the United States (Targoff, Curr Probl Dermatol. 199.1, 3:131-180). Idiopathic inflammatory myopathy is associated with significant morbidity and mortality, with up to half of affected adults noted to have suffered significant impairment (Gottdiener et al., Am J Cardiol. 1978, 41:1141-49). Miller (Rheum Dis Clin North Am. 1994, 20:811-826 and Arthritis and Allied Conditions, Ch. 75, Eds. Koopman and Moreland, Lippincott Williams and Wilkins, 2005) sets out five groups of criteria used to diagnose IIM, i.e., Idiopathic Inflammatory Myopathy Criteria (IIMC) assessment, including muscle weakness, muscle biopsy evidence of degeneration, elevation of serum levels of muscle-associated enzymes, electromagnetic triad of myopathy, evidence of rashes in dermatomyositis, and also includes evidence of autoantibodies as a secondary criteria.

IIM associated factors, including muscle-associated enzymes and autoantibodies include, but are not limited to, creatine kinase (CK), lactate dehydrogenase, aldolase, C-reactive protein, aspartate aminotransferase (AST), alanine aminotransferase (ALT), and antinuclear autoantibody (ANA), myositis-specific antibodies (MSA), and antibody to extractable nuclear antigens.

A "binding molecule" according to the invention can be, for example, a protein (a "protein" may be a polypeptide or peptide), nucleic acid, carbohydrate, lipid, or small molecule compound that binds to a target. A type of proteinaceous binding molecule contemplated by the invention is an antibody or an antibody fragment that retains binding activity. A binding molecule may be modified according to methods standard in the art to improve its binding affinity, diminish its immunogenicity, alter its effector functions and/or improve its availability in the body of an individual. Such modifications may include, for example, amino acid sequence modifications or expression as a fusion protein. Such fusion proteins are also binding molecules according to the invention. An exemplary binding molecule of the invention is a small modular immunopharmaceutical (SMIP™).

A binding molecule that is "specific" for a target binds to that target with a greater affinity than any other target. For example, a CD20-specific binding molecule binds to CD20 with a greater affinity than to any other target. Binding molecules of the invention may have affinities for their targets of a Ka of greater than or equal to about 10⁴ M⁻¹, preferably of greater than or equal to about 10⁵ M⁻¹, more preferably of greater than or equal to about 10⁶ M⁻¹ and still more preferably of greater than or equal to about 10⁷ M⁻¹. Affinities of even greater than about 10⁷ M⁻¹ are still more preferred, such as affinities equal to or greater than about 10⁷ M⁻¹, about 10⁸ M⁻¹, and about 10⁹ M⁻¹, and about 10¹⁰ M⁻¹. Affinities of binding molecules according to the present invention can be readily determined using conventional techniques, for example those described by Scatchard et al., Ann. N.Y. Acad. Sci, 51:660 (1949). In one embodiment, the CD20-specific binding molecule has an affinity for CD20 in the range of 1 nM to 30 nM.

In an additional aspect, the CD20-specific binding molecule has a half-life of 7 to 30 days *in vivo*.

Methods for making small, modular immunopharmaceuticals (SMIPs) have been described previously in co-owned U.S. application no. 10/627,556 and US Patent Publications 2003/133939, 2003/0118592, and 2005/0136049, which are incorporated herein by reference in their entirety. SMIPs are novel binding domain-immunoglobulin fusion proteins that feature a binding domain for a cognate structure such as an antigen, a counterreceptor or the like; an IgG1, IgA or IgE hinge region polypeptide or a mutant IgG1 hinge region polypeptide having either zero, one or two cysteine residues; and immunoglobulin CH2 and CH3 domains. In one embodiment, the binding domain molecule has one or two cysteine residues. In a related embodiment, it is contemplated that when the binding domain molecule comprises two cysteine residues, the first cysteine, which is typically involved in binding between the heavy chain and light chain variable regions, is not deleted or substituted with an amino acid.

The binding domain of molecules useful in methods of the invention are contemplated as having one or more binding regions, such as variable light chain and variable heavy chain binding regions derived from one or more immunoglobulins superfamily members, such as an immunoglobulin. These regions, moreover, are typically separated by linker peptides, which may be any linker peptide known in the art to be compatible with domain or region joinder in a binding molecule. Exemplary linkers are linkers based on the Gly₄Ser linker motif, such as (Gly₄Ser)ₙ, where n=3-5. The molecules for use in the methods of the invention also contain sufficient amino acid sequence derived from a constant region of an immunoglobulin to provide an effector function, preferably ADCC and/or CDC. Thus, the molecules will have a sequence derived from a CH2 domain of an immunoglobulin or CH2 and CH3 domains derived from one or more immunoglobulins, SMIPs are capable of ADCC and/or CDC but are compromised in their ability to form disulfide-linked multimers.

Exemplary CD20-specific SMIPs include SMIPs derived from the anti-CD20 monoclonal antibody 2H7 described in US Patent Publ. Nos. 2003133939 and 20030118592. The SMIPs include 2H7scFv-Ig or a derivative thereof. Derivatives includes CytoxB-MHWTG1C, which has a human IgG1 Fc domain and a mutant IgG1 hinge domain; CytoxB-MHMG1C, which comprises a mutated Fc domain; MG1H/MG1C, which comprises an Fc receptor with a mutated leucine residue 234; CytoxB-IgAHWTHG1C, comprising a portion of the human IgA hinge fused to human Fc domain; 2H7 seFv-Ilama IgG1, comprising the Ilama IgG1 hinge and CH2CH3 regions, 2H7 scFv-Ilama IgG2, comprising the Ilama IgG2 hinge and CH2CH3 regions; 2H7 scFv-Ilama IgG3, comprising the Ilama IgG3 hinge and CH2CH3 regions.

2H7scFv-Ig derivatives also include 2H7 scFv mutants with point mutations in the hinge region. 2H7 scFv MTH (SSS) WTCH2CH3, in which all three cysteine residues in the connection or hinge regions are mutated to serine residues, and wild type CH2 and CH3 domains; 2H7 scFv MTH (SSC), in which the first two cysteine residues were substituted with serine residues; 2H7 scFv MTH (SCS), in which the first and third cysteines were substituted with serine residues; 2H7 scFv MTH (CSS) WTCH2CH3, in which cysteine residues were substituted at the second and third positions with serine; 2H7 scFv VH11SER IgG MTH (SSS) WTCH2CH3, in which the leucine at position 11 in the heavy chain variable region is substituted with serine; 2H7 scFv IgA hinge-IgG1 CH2-CH3, comprising an IgA hinge region and WT IgG1 domains; 2H7 scFv IgA hinge-CH2-CH3, comprising IgA hinge, CH2-CH3 regions; 2H7 IgAWH IgACH2-T4CH3, comprising an IgA hinge, a wild type IgA CH2 and a truncated IgA CH3 domain lacking the 4 carboxy amino acids GTCY.

Derivatives with mutations in the IgG CH3 region include 2H7 scFv MTH WTCH2MTCH3 Y405, in which phenylalanine residue at position 405 (numbering according to Kabat *et al. supra*) was substituted with tyrosine; 2H7 scFv MTH WTCH2 MTCH3 A405, in which phenylalanine position at 405 was substituted with an alanine; scFv MTH WTCH2 MTCH3 A407, in which tyrosine residue at position 407 was substituted with an alanine; scFv MTH WTCH2 MTCH3 Y405A407, comprising the two mutations; and scFv MTH WTCH2 MTCH3 A405A407 comprising two mutations.

21-17 scFv MTH (CCS) WTCH2CH3 is a construct with the third cysteine residue in the IgG1 hinge region substituted with a serine residue. The 2H7 scFv IgG MTH (SSS) MTCH2WTCH3 SMIP comprises mutant hinge (MT (SSS)) and a mutant CH2 domain in which the proline at residue 238 (according to Ward *et al.*) was substituted with a serine.

2H7scFv-lg derivatives also include 2H7 scFv mutants with point mutations in the variable heavy chain region The following constructs all comprises mutations in which the leucine at position 11 in the heavy chain variable region is substituted with serine: 2H7 scFv VH11SER IgG MTH (SSS-S) WTCH2CH3, 2H7scFv VHL11S (CSS-S) H WCH2 WCH3, comprising a mutated hinge region as set out above; 2H7scFv VHL11S (CSC-S) H WGH2 WCH3 comprising a mutated hinge region as set out above; 2H7 scFv VHL11S IgAH IgACH2 T4CH3, comprises the IgA hinge, WT igA CH2 and truncated IgA CH3; 2H7 scfv VHL11S IgECH2 CH3 CH4, comprising the IgE CH 2-4 regions; 2H7 VHL11S scFv (SSS-S) IgECH3CH4, comprising a mutated hinge region as set out above and IgE CH3 and CH4 regions; 2H7 scfv VH L11S mlgE CH2 CH3 CH4, comprises mouse IgE regions; 2H7 scFv VH L11S mlgAH WIGACH2 T4CH3 comprises the mutations described above and a mouse IgA constant region consisting of a wild type CH2 region and a mutated CH3 region; 2H7 scFv VH L11S (SSS-S) H K322S CH2 WCH3 comprises a mutation in the human IgG1 CH2 region at residue 322, where lysine was changed to serine; 2H7 scFv VH L11S (CSS-S) H K322S CH2 WCH3 comprises a mutated hinge region as described above, and a mutated CH2 region as previously described; 2H7 scFv VH L11S (SSS-S) H P331S CH2 WCH3, comprises a mutated hinge region as described above, and a mutated CH2 region in which proline at residue 331 was changed to a serine; 2H7 scFv VH L11S (CSS-S) H P331S CH2 WCH3 comprises a mutated hinge region and a proline to serine mutation at residue 331 in the CH2 region; 2H7 scFv VH L11S (SSS-S) H T256N CH2 WCH3, comprises a mutated hinge region and a threonine to asparagine mutation at residue 256 in the CH2 region; 2H7 scFv VH L11S (SSS-S) H RTPE/QNAK (255-258) CH2 WCH3, comprises a mutated hinge region and a series of mutations in which residues 255-258 have been mutated from arginine, threonine, proline, glutamic acid to glutamine, asparagines, alanine and lysine, respectively; 2H7 scFv VH L11S (SSS-S) H K290Q CH2 WCH3, comprises a mutated hinge regions and a lysine to glutamine change at position 290; 2H7 scFv VH L11S (SSS-S) H A339P CH2 WCH3, comprises a mutated hinge region and an alanine to proline change at position 339.

SMIP 2H7 scfv (SSS-S) H P238SCH2 WCH3, comprises a mutated hinge region and an proline to serine change at position 238 in CH2, which is the same as 2H7 scFv IgG MTH (SSS) MTCH2WTCH3. 2H7 scFv IgAH IGAHCH2 T18CH3 comprises a wild type IgA hinge and CH2 region and a CH3 region with an 18 amino acid truncation at the carboxy end.

It is contemplated that a binding molecule of the invention may comprise a native or engineered extracellular domain from another protein which improves the binding molecule activity. In one embodiment, the extracellular domain is selected from the group consisting of CD154 and CTLA4.

In one aspect of the invention, the CD20-specific binding molecule is administered as a pharmaceutical composition. To administer the CD20-specific binding molecule to humans or test animals, it is preferable to formulate the binding molecule in a composition comprising one or more pharmaceutically acceptable carriers. The phrase "pharmaceutically or pharmacologically acceptable" refer to molecular entities and compositions that do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like.

In addition, compounds may form solvates with water or common organic solvents. Such solvates are contemplated as well.

The CD20-specific binding molecule compositions may be administered orally, topically, transdermally, parenterally, by inhalation spray, vaginally, rectally, or by intracranial injection. The term parenteral as used herein incudes subcutaneous injections, intravenous, intramuscular, intracisternal injection, or infusion techniques, Administration by intravenous, intradermal, intramusclar, intramammary, intraperitoneal, intrathecal, retrobulbar, intrapulmonary injection and or surgical implantation at a particular site is contemplated as well. Generally, compositions are essentially free of pyrogens, as well as other impurities that could be harmful to the recipient. Injection, especially intravenous, are preferred.

Pharmaceutical compositions of the present invention containing a CD20-specific binding molecule used in a method of the invention may contain pharmaceutically acceptable carriers or additives depending on the route of administration. Examples of such carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, glycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the line, Additives used are chosen from, but not limited to, the above or combinations thereof, as appropriate, depending on the dosage form of the present invention.

Formulation of the pharmaceutical composition will vary according to the route of administration selected (e.g., solution, emulsion). An appropriate composition comprising the antibody to be administered can be prepared in a physiologically acceptable vehicle or carrier. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. Intravenous vehicles can include various additives, preservatives, or fluid, nutrient or electrolyte replenishers

A variety of aqueous carriers, waster, buffered water, 0.4% saline, 0.3% glycine, or aqueous suspensions may contain the active compound in admixture with excipients suitable for the manufacture of aqueous suspensions, Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaothyl-eneoxycotanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate, The aqueous suspensions may also contain one or more preservatives, for example ethyl, or n-propyl, p-hydroxybenzoate.

The CD20-specific binding molecule composition can be lyophilized for storage and reconstituted in a suitable carrier prior to use, This technique has been shown to be effective with conventional immunoglobulins. Any suitable lyophilization and reconstitution techniques can be employed. It will be appreciated by those skilled in the art that lyophilization and reconstitution can lead to varying degrees of antibody activity loss and that use levels may have to be adjusted to compensate.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above.

The concentration of CD20-specific binding molecule in these formulations can vary widely, for example from less than about 0.5%, usually at or at least about 1 % to as much as 15 or 20% by weight and will be selected primarily based on fluid volumes, viscosities, etc., in accordance with the particular mode of administration selected. Thus, a typical pharmaceutical composition for parenteral injection could be made up to contain 1 ml sterile buffered water, and 50 mg of antibody. A typical composition for intravenous infusion could be made up to contain 250 ml of sterile Ringer's solution, and 150 mg of antibody. Actual methods for preparing parenterally administrable compositions will be known or apparent to those skilled in the art and are described in more detail in, for example, Remington's Pharmaceutical Science, 15th ed., Mack Publishing Company, Easton, Pa. (1980). An effective dosage of antibody is within the range of 0.01 mg to 1000 mg per kg of body weight per administration.

The pharmaceutical compositions may be in the form of a sterile injectable aqueous, oleaginous suspension, dispersions or sterile powders for the extemporaneous preparation of sterile injectable solutions or dispersions. The suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. The carrier can be a solvent or dispersion medium containing, for example, water, ethanol, polyol (for example, glycerol, propylene glycol, and liquid polyethylene glycol, and the like), suitable mixtures thereof, vegetable oils, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

In all cases the form must be sterile and must be fluid to the extent that easy syringability exists. The proper fluidity can be maintained, for example, by the use of a coating, such as lecithin, by the maintenance of the required particle size in the case of dispersion and by the use of surfactants. It must be stable under the conditions of manufacture and storage and must be preserved against the contaminating action of microorganisms, such as bacteria and fungi. The prevention of the action of microorganisms can be brought about by various antibacterial and antifungal agents, for example, parabens, chlorobutanol, phenol, sorbic acid, thimerosal, and the like. In many cases, it will be desirable to include isotonic agents, for example, sugars or sodium chloride. Prolonged absorption of the injectable compositions can be brought about by the use in the compositions of agents delaying absorption, for example, aluminum monostearate and gelatin.

Compositions useful for administration may be formulated with uptake or absorption enhancers to increase their efficacy, Such enhancer include for example, salicylate, glycocholate/linoleate, glycholate, aprotinin, bacitracin, SDS, caprate and the like. See, *e.g.*, Fix (J. Pharm. Sci., 85:1282-1285, 1996) and Oliyai and Stella (Ann. Rev, Pharmacol. Toxicol., 32:521-544,1983).

In addition, the properties of hydrophilicity and hydrophobicity of the compositions contemplated for use in the invention are well balanced, thereby enhancing their utility for both *in vitro* and especially *in vivo* uses, while other compositions lacking such balance are of substantially less utility. Specifically, compositions contemplated for use in the invention have an appropriate degree of solubility in aqueous media which permits absorption and bioavailability in the body, while also having a degree of solubility in lipids which permits the compounds to traverse the cell membrane to a putative site of action. Thus, antibody compositions contemplated are maximally effective when they can be delivered to the site of target antigen activity.

In one aspect, methods of the invention include a step of administration of a pharmaceutical composition.

Methods of the invention are performed using any medically-accepted means for introducing a therapeutic directly or indirectly into a mammalian subject, including but not limited to injections, oral ingestion, iritranasel, topical, transdermal, parenteral, inhalation spray, vaginal, or rectal administration. The term parenteral as used herein includes subcutaneous, intravenous, intramuscular, and intracisternal injections, as well as catheter or infusion techniques. Administration by, intradermal, intramammary, intraperitoneal, intrathecal, retrobulbar, intrapulmonary injection and or surgical implantation at a particular site is contemplated as well.

In one embodiment, administration is performed at the site of a cancer or affected tissue needing treatment by direct injection into the site or via a sustained delivery or sustained release mechanism, which can deliver the formulation internally. For example, biodegradable microspheres or capsules or other biodegradable polymer configurations capable of sustained delivery of a composition (*e.g.*, a soluble polypeptide, antibody, or small molecule) can be included in the formulations of the invention implanted near the cancer.

Therapeutic compositions may also be delivered to the patient at multiple sites. The multiple administrations may be rendered simultaneously or may be administered over a continuous period of time,

Also contemplated in the present invention is the administration of a binding molecule composition in conjunction with a second agent. Second agents contemplated by the invention are listed in the paragraphs below.

A second agent may be a B-cell-associated molecule. Other B-cell-associated molecules contemplated by the invention include binding molecules which bind to B-cell surface molecules that are not CD20. B-cell-associated molecules include, but are not limited to, CD19 (B-lymphocyte antigen CD19, also referred to as B-lymphocyte surface antigen B4, or Leu-12), CD21, CD22 (B-cell receptor CD22, also referred to as Leu-14, B-lymphocyte cell adhesion molecule, or BL-CAM), CD23, CD37, CD40 (B-cell surface antigen CD40, also referred to as Tumor Necrosis Factor receptor superfamily member 5, CD40L receptor, or Bp50), CD80 (T lymphocyte activation antigen CD80, also referred to as Activation B7-1 antigen, B7, B7-1, or BB1), CD86 (T lymphocyte activation antigen CD86, also referred to as Activation B7-2 antigen, B70, FUN-1, or BU63), CD137 (also referred to as Tumor Necrosis Factor receptor superfamily member 9), CD152 (also referred to as cytotoxic T-lymphocyte protein 4 or CTLA-4), L6 (Tumor-associated antigen L6, also referred to as Transmembrane 4 superfamily member 1, Membrane component surface marker 1, or M3S1), CD30 (lymphocyte activation antigen CD30, also referred to as Tumor Necrosis Factor receptor superfamily member 8, CD30L receptor, or Ki-1), CD50 (also referred to as Intercellular adhesion molecule-3 (ICAM3), or ICAM-R), CD54 (also referred to as intercellular adhesion molecule-1 (ICAM1), or Major group rhinovirus receptor), B7-H1 (ligand for an immunoinhibitory receptor expressed by activated T cells, B-cells, and myeloid cells, also referred to as PD-L1; see Dong, et al., "B7-H1, a third member of the B7 family, co-stimulates T-cell proliferation and interleukin-10 secretion," Nat. Med. 1999, 5:1365-1369), CD134 (also referred to as Tumor Necrosis Factor receptor superfamily member 4, OX40, OX40L receptor, ACT35 antigen, or TAX-transcriptionally activated glycoprotein 1 receptor). 41BB (4-1BB ligand receptor, T-cell antigen 4-1BB, or T-cell antigen ILA), CD153 (also referred to as Tumor Necrosis Factor ligand superfamily member 8, CD30 ligand, or CD30-L), CD154 (also referred to as Tumor Necrosis Factor ligand superfamily member 5, TNF-related activation protein, TRAP, or T cell antigen Gp39) and Toll receptors, The above list of construct targets and/or target antigens is exemplary only and is not exhaustive.

Cytokines and growth factors contemplated by the invention as second agents include, without limitation, one or more ofTNF, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IFN, G-CSF, Meg-CSF, GM-CSF, thrombopoietin, stem cell factor, and erythropoietin, Pharmaceutical compositions in accordance with the invention may also include other known angiopoietins, for example Ang-1, Ang-2, Ang-4, Ang-Y, and/or the human angiopoietin-like polypeptide, and/or vascular endothelial growth factor (VEGF). Growth factors for use in pharmaceutical compositions of the invention include angiogenin, bone morphogenic protein-1, bone morphogenic protein-2, bone morphogenic protein-3, bone morphogenic protein-4, bone morphogenic protein-5, bone morphogenic protein-6, bone morphogenic proteins-7, bone morphogenic protein-8, bone morphogenic protein-9, bone morphogenic protein-10, bone morphogenic protein-11, bone morphogenic protein-12, bone morphogenic protein-13, bone morphogenic protein-14, bone morphogenic protein-15, bone morphogenic protein receptor IA, bone morphogenic protein receptor IB, brain derived neurotrophic factor, ciliary neutrophic factor, ciliary neutrophic factor receptor α, cytokine-induced neutrophil chemotactic factor 1, cytokine-induced neutrophil chemotactic factor 2α, cytokine induced neutrophil chemotactic factor 2β, β endothelial cell growth factor, endothelin 1, epidermal growth factor, epithelial-derived neutrophil attractant, fibroblast growth factor 4, fibroblast growth factor 5, fibroblast growth factor 6, fibroblast growth factor 7, fibroblast growth factor 8, fibroblast growth factor 8b, fibroblast growth factor 8c, fibroblast growth factor 9, fibroblast growth factor 10, fibroblast growth factor acidic, fibroblast growth factor basic, glial cell line-derived neutrophic factor receptor α1, glial cell line-derived neutrophic factor receptor a2, growth related protein, growth related protein α, growth related protein β, growth related proteins, heparin binding epidermal growth factor, hepatocyte growth factor, hepatocyte growth factor receptor, insulin-like growth factor I, insulin-like growth factor receptor, insulin-like growth factor II, insulin-like growth factor binding protein, keratinocyte growth factor, leukemia inhibitory factor, leukemia inhibitory factor receptor α, nerve growth factor, nerve growth factor receptor, neurotrophin-3, neurotrophin-4, placenta growth factor, placenta growth factor 2, platelet derived endothelial cell growth factor, platelet derived growth factor, platelet derived growth factor A chain, platelet derived growth factor AA, platelet derived growth factor AB, platelet derived growth factor B chain, platelet derived growth factor BB, platelet derived growth factor receptor α, platelet derived growth factor receptor β, pre-B-cell growth stimulating factor, stem cell factor, stem cell factor receptor, transforming growth factor α, transforming growth factor β, transforming growth factor β1, transforming growth factor β1.2, transforming growth factor β2, transforming growth factor β3, transforming growth factor β5, latent transforming growth factor β1, transforming growth factor β binding protein I, transforming growth factor β binding protein II, transforming growth factor β binding protein III, tumor necrosis factor receptor type I, tumor necrosis factor receptor type II, urokinase-type plasminogen activator receptor, vascular endothelial growth factor, and chimeric proteins and biologically or immunologically active fragments thereof.

Examples of chemotherapeutic agents contemplated as second agents include, but are not limited to alkylating agents, such as nitrogen mustards (e.g., mechlorethamine, cyclophosphamide, ifosfamide, melphalan, and chlorambucil); nitrosoureas (e.g., carmustine (BCNU), lomustine (CCNU), and semustine (methyl-CCNU)); ethylenimines and methyl-melamines (e.g., triethylenemelamine (TEM), triethylene thiophosphoramide (thiotepa), and hexamethylmelamine (HMM, altretamine)); alkyl sulfonates (e.g., buslfan); and triazines (e.g., dacabazine (DTIC)); antimetabolites, such as folic acid analogs (e.g., methotrexate, trimetrexate, and pemetrexed (multi-targeted antifotate)); pyrimidine analogs (such as 5-fluorouracil (5-FU), fluorodeoxyuridine, gemcitabine, cytosine arabinoside (AraC, cytarabine), 5-azacytidine, and 2,2'-difluorodeoxycytidine); and purine analogs (e.g., 6-mercaptopurine, 6-thioguanine, azathioprine, 2'-deoxycoformycin (pentostatin), erythrohydroxynonyladenine (EHNA), fludarabine phosphate, 2-chlorodeoxyadenosine (cladribine, 2-CdA)); Type I topoisomerase inhibitors such as camptothecin (CPT), topotecan, and irinotecan; certain natural products, such as epipodophylotoxins (e.g., etoposide and teniposide); and vinca alkaloids (e.g., vinblastine, vincristine, and vinorelbine); anti-tumor antibiotics such as actinomycin D, doxorubicin, and bleomycin; certain radiosensitizers such as 5-bromodeozyuridine, 5-iododeoxyuridine, and bromodeoxycytidine; platinum coordination complexes such as cisplatin, carboplatin, and oxaliplatin; substituted ureas, such as hydroxyurea; and methylhydrazine derivatives such as N-methylhydrazine (MIH) and procarbazine.

Non-limiting examples of chemotherapeutic agents, radiotherapeutic agents and other active and ancillary agents are also shown in Table 1.

**TABLE 1**

| **Alkylating agents** | **Natural products** |
|---|---|
| Nitrogen mustards | Antimitotic drugs |
| mechlorethamine | |
| cyclophosphamide | |
| ifosfamide | Taxanes |
| melphalan | paclitaxel |
| chlorambucil | Vinca alkaloids |
| | vinblastine (VLB) |
| Nitrosoureas | vincristine |
| carmustine (BCNU) | vinorelbine |
| lomustine (CCNU) | Taxotere® (docetaxel) |
| semustine (methyl-CCNU) | estramustine |
| | estramustine phosphate |
| Ethylenimine/Methyl-melamine | |
| thriethylenemelamine (TEM) | Epipodophylotoxins |
| triethylene thiophosphoramide | etoposide |
| (thiotepa) | teniposide |
| hexamethylmelamine | |
| (HMM, attretamine) | Antibiotics |
| | actimomycin D |
| Alkyl sulfonates | daunomycin (rubido)-mycin) |
| busulfan | doxorubicin (adria-mycin) |
| | mitoxantroneidarubicin |
| Triazines | bleomycin |
| dacarbazine (DTIC) | splicamycin (mithramycin) |
| | mitomycinC |
| **Antimetabolites** | dactinomycin |
| Folic Acid analogs | aphidicolin |
| methotrexate | |
| Trimetrexate | Enzymes |
| Pemetrexed | L-asparaginase |
| (Multi-targeted antifolate) | L-arginase |
| Pyrimidine analogs | **Radiosensitizers** |
| 5-fluorouracil | metronidazole |
| fluorodeoxyuridine | misonidazole |
| gemcitabine | desmethylmisonidazole |
| cytosine arabinoside | pimonidazole |
| (AraC, cytarabine) | etanidazole |
| 5-azacytidine | nimorazole |
| 2,2'- difluorodeoxy-cytidine | RSU 1069 |
| | EO9 |
| Purine analogs | RB 6145 |
| 6-mercaptopurine | SR4233 |
| 6-thioguanine | nicotinamide |
| azathioprine | 5-bromodeozyuridine |
| 2'-deoxycoformycin | 5-iododeoxyuridine |
| (pentostatin) | bromodeoxycytidine |
| erythrohydroxynonyl-adenine (EHNA) | |
| fludarabine phosphate | |
| 2-chlorodeoxyadenosine | **Miscellaneous agents** |
| (cladribine, 2-CdA) | Platinum coordination complexes |
| | cisplatin |
| | Carboplatin |
| **Type I Topoisomerase Inhibitors** | oxaliplatin |
| camptothecin | Anthracenedione |
| topotecan | mitoxantrone |
| irinotecan | |
| | Substituted urea |
| **Biological response modifiers** G-CSF | hydroxyurea |
| GM-CSF | Methylhydrazine derivatives |
| | N-methylhydrazine (MIH) |
| **Differentiation Agents** | procarbazine |
| retinoic acid derivatives | |
| | Adrenocortical suppressant |
| **Hormones and antagonists** | mitotane (*o*,*p*'- DDD) |
| Adrenocorticosteroids/ antogonists | ainoglutethimide |
| prednisone and equivalents | |
| dexamethasone | **Cytokines** |
| ainoglutethimide | interferon (α, β, γ) |
| Progestins | interleukin-2 |
| hydroxyprogesterone caproate | **Photosensitizers** |
| medroxyprogesterone acetate | hematoporphyrin derivatives |
| megestrol acetate | Photofrin® |
| | benzoporphyrin derivatives |
| Estrogens | Npe6 |
| diethylstilbestrol | tin etioporphyrin (SnET2) |
| ethynyl estradiol/ equivalents | pheoboride-a |
| | bacteriochlorophyll-a |
| Antiestrogen | naphthalocyanines |
| tamoxifen | phthalocyanines |
| | zinc phthalocyanines |
| Androgens | |
| testosterone propionate | **Radiation** |
| fluoxymesterone/equivalents | X-ray |
| | ultraviolet light |
| Antiandrogens | gamma radiation |
| flutamide | visible light |
| gonadotropin-releasing | infrared radiation |
| hormone analogs | microwave radiation |
| leuprolide | |
| Nonsteroidal antiandrogens | |
| Flutamide | |

Second agents contemplated by the invention for treatment of autoimmune diseases are referred to as immunosuppressive agents, which act to suppress or mask the immune system of the individual being treated. Immunosuppressive agents include, for example, non-steroidal anti-inflammatory drugs (NSAIDs), analgesiscs, glucocorticoids, disease-modifying antirheumatic drugs (DMARDs) for the treatment of arthritis, or biologic response modifiers. Compositions in the DMARD description are also useful in the treatment of many other autoimmune diseases aside from RA.

Exemplary NSAIDs are chosen from the group consisting of ibuprofen, naproxen, naproxen sodium, Cox-2 inhibitors such as Vioxx and Celebrex, and sialylates. Exemplary analgesics are chosen from the group consisting of acetaminophen, oxycodone, tramadol of proporxyphene hygrochloride. Exemplary glucocorticoids are chosen from the group consisting of cortisone, dexamethosone, hydrocortisone, methylprednisolone, prednisolone, or prednisone. Exemplary biological response modifiers include, but are not limited to, molecules directed against cell surface markers (e.g., CD4, CD5, CTLA4, etc.), abatacept, cytokine inhibitors, such as the TNF antagonists (e.g. etanercept (Enbrel), adalimumab (Humira), and infliximab (Remicade)), chemokine inhibitors and adhesion molecule inhibitors. The biological response modifiers include monoclonal antibodies as well as recombinant forms of molecules, Exemplary DMARDs include, but are not limited to, azathioprine, cyclophosphamide, cyclosporine, methotrexate, penicillamine, leflunomide, sulfasalazine, hydroxychloroquine, Gold [oral (auranofin) and intramuscular] and minocycline.

It is contemplated that the CD20-specific binding molecule composition and the second agent may be given simultaneously in the same formulation. Alternatively, the agents are administered in a separate formulation and administered concurrently, with concurrently referring to agents given within 30 minutes of each other.

In another aspect, the second agent is administered prior to administration of the CD20-specific binding molecule composition. Prior administration refers to administration of the second agent within the range of one week prior to treatment with the antibody, up to 30 minutes before administration of the antibody. It is further contemplated that the second agent is administered subsequent to administration of the SMIP composition. Subsequent administration is meant to describe administration from 30 minutes after antibody treatment up to one week after antibody administration.

It is further contemplated that when the CD20-specific binding molecule is administered in combination with a second agent, wherein the second agent is a cytokine or growth factor, or a chemotherapeutic agent, the administration also includes use of a radiotherapeutic agent or radiation therapy. The radiation therapy administered in combination with an antibody composition is administered as determined by the treating physician, and at doses typically given to patients being treated for cancer.

The amounts of CD20-specific binding molecule composition in a given dosage will vary according to the size of the individual to whom the therapy is being administered as well as the characteristics of the disorder being treated. In exemplary treatments, it may be necessary to administer about 1 mg/day, about 5 mg/day, about 10 mg/day, about 20 mg/day, about 50 mg/day, about 75 mg/day, about 100 mg/day, about 150 mg/day, about 200 mg/day, about 250 mg/day, about 400 mg/day, about 500 mg/day, about 800 mg/day, about 1000 mg/day, about 1600 mg/day or about 2000 mg/day. The doses may also be administered based on weight of the patient, at a dose of 0.01 to 50 mg/kg. In a related embodiment, the CD20-specific binding molecule may be administered in a dose range of 0.015 to 30 mg/kg. In an additional embodiment, the CD20-specific binding molecule is administered in a dose of about 0.015, about 0.05, about 0.15, about 0.5, about 1.5, about 5, about 15 or about 30 mg/kg.

Standard dose-response studies, first in animal models and then in clinical testing, reveal optimal dosages for particular diseases and patient populations.

The administration of the CD20-specific binding molecule composition decreases or reduces the B-cell population by at least about 20% after a single dose of treatment. In one embodiment, the B-cell population is decreased or reduced by at least about 20, about 30, about 40, about 50, about 60, about 70, about 80, about 90 or about 100%. B-cell depletion is defined as a decrease in absolute B-cell count below the lower limit of the normal range. B-cell recovery is defined as a return of absolute B-cell count to either of the following: 1) 70% of subject's baseline value; or 2) normal range.

The administration of CD20-specific binding molecules also results in enhanced apoptosis in particular B-cell subsets. Apoptosis refers to the induction of programmed cell death of a cell, manifested and assessed by DNA fragmentation, cell shrinkage, cell fragmentation, formation of membrane vesicles, or alteration of membrane lipid composition as assessed by annexin V staining.

Further, the administration of CD20-specific binding molecules results in desired clinical effects in the disease or disorder being treated. For example, in patients affected by rheumatoid arthritis, administration of CD20 molecules improves the patient's condition by a clinically significant amount [e.g., achieves the American College of Rheumatology Preliminary Detection of Improvement (ACR20)], and/or an improvement of 20% in tender and swollen joint and 20% improvement in 3/5 remaining ACR measures (Felson et al., Arthritis Rheum. 1995, 38:727-35). Biological measures for improvement in an RA patient after administration of CD20-specific binding molecule include measurement of changes in cytokine levels, measured via protein or RNA levels. Cytokines of interest include, but are not limited to, TNF-α, IL-1, interferons, Blys, and APRIL. Cytokine changes may be due to reduced B cell numbers or decreased activated T cells. In RA patients, markers relevant to bone turnover (bone resorption or erosion) are measured before and after administration of CD20-specific binding molecules. Relevant markers include, but are not limited to, alkaline phosphatase, osteocalcin, collagen breakdown fragments, hydroxyproline, tartrate-resistant acid phosphotase, and RANK ligand (RANKL). Other readouts relevant to the improvement of RA include measurement of C reactive protein (CRP) levels, erythrocyte sedimentation rate (ESR), rheumatoid factor, CCP (cyclic citrullinated peptide) antibodies and assessment of systemic B cell levels and lymphocyte count via flow cytometry. Specific factors can also be measured from the synovium of RA patients, including assessment of B cell levels in synovium from synovium biopsy, levels of RANKL and other bone factors and cytokines set out above.

In a related aspect, the effects of CD20-specific binding molecule administration on other diseases is measured according to standards known in the art. For example, it is contemplated that Crohn's disease patients receiving CD20-specific binding molecules achieve an improvement in Crohn's Disease Activity Index (CDAI) in the range of about 50 to about 70 units, wherein remission is at 150 units (Simonis et al, Scand. J Gastroent. 1998, 33:283-8). A score of 150 or 200 is considered normal, while a score of 450 is considered a severe disease score. It is further desired that administration of the CD20-specific binding molecule results in a reduction in perinuclear anti-neutrophil antibody (pANCA) and anti-Saccharomyces cervisiae antibody (ASCA) in individuals affected by inflammatory bowel disease.

It is further contemplated that adult and juvenile myositis patients receiving CD20-specific binding molecules achieve an improvement in core set of evaluations, such as 3 out of 6 of the core set measured improved by approximately 20%, with not more than 2 of the core measurements worse by approximately 25% (see Rider et al., Arthritis Rheum. 2004, 50:2281-90).

It is further contemplated that SLE patients receiving CD20-specific binding molecules achieve an improvement in Systemic Lupus Activity Measure (SLAM) or SLE Disease Activity Index (SLEDAI) score of at least 1 point (Gladman et al, J Rheumatol 1994, 21:1468-71) (Tan et al., Arthritis Rheum, 1982, 25:1271-7). A SLAM score of >5, or SLEDAI score >2, is considered clinically active disease. A response to treatment may be defined as improvement or stabilization over the in 2 disease activity measures (the SLE Disease Activity Index [SLEDAI] and the Systemic Lupus Activity Measure) and 2 quality of life measures (patient's global assessment and the Krupp Fatigue Severity Scale) (Petri et al., Arthritis Rheum. 2004, 50:2858-68.) It is further desired that administration of the CD20-specific binding molecule to SLE patients results in a reduction in anti-double-stranded DNA antibodies. Alternatively, improvement may be gauged using the British Isles Lupus Assessment Group Criteria (BILAG).

It is further contemplated that multiple sclerosis patients receiving CD20-specific binding molecules achieve an improvement in clinical score on the Kurtzke Expanded Disability status scale (EDSS) (Kurtzke, F., Neurology 1983, 33:1444-52) of at least 0.5, or a delay in worsening of clinical disease of at least 1.0 on the Kurtzke scale (Rudick et al., Neurology 1997, 49:358-63).

It is further contemplated that patients suffering from IIM receiving CD20-specific binding molecules achieve a reduction in at least one of five criteria set out in the Idiopathic Inflammatory Myopathy Criteria (IIMC) assessment (Miller, F., *supra*). It is further contemplated that administration of the CD20-specific binding molecule to IIM patients results in a reduction in IIM-associated factors selected from the group consisting of creatine kinase (CK), lactate dehydrogenase, aldolase, C-reactive protein, aspartate aminotransferase (AST), alanine aminotransferase (ALT), and antinuclear autoantibody (ANA), myositis-specific antibodies (MSA), and antibody to extractable nuclear antigens. Alternatively, patients meeting 3 out of 6 of the criteria set out in Rider et al., Arthritis Rheum. 2004, 50:2281-90, may be the subject of treatment according to the invention, with worsening in no more than 2 criteria.

In a still further embodiment, patients suffering from a B cell cancer receive a CD20-specific binding molecule and demonstrate an overall beneficial response to the CD20-specific binding molecule, based on clinical criteria well-known and commonly used in the art, and as described below, such as a decrease in tumor size, decrease in tumor number and/or an improvement in disease symptoms.

For example, the U.S. National Cancer Institute (NCI) has divided some of the classes of cancers into the clinical categories of "indolent" and "aggressive" lymphomas, indolent lymphomas include follicular cell lymphomas, separated into cytology "grades," diffuse small lymphocytic lymphoma/chronic lymphocytic leukemia (CLL), lymphoplasmacytoid/Waldenstrom's Macroglobulinemia, Marginal zone lymphoma and Hairy cell leukemia. Aggressive lymphomas include diffuse mixed and large cell lymphoma, Burkitt's lymphoma/diffuse small non-cleaved cell lymphoma, Lymphoblastic lymphoma, Mantle cell lymphoma and AIDS-related lymphoma. In some cases, the International Prognostic Index (IPI) is used in cases of aggressive and follicular lymphoma. Factors to consider in the IPI include Age (<60 years of age versus >60 years of age), serum lactate dehydrogenase (levels normal versus elevated), performance status (0 or 1 versus 2-4) (see definition below), disease stage (I or II versus III or IV), and extranodal site involvement (0 or 1 versus 2-4). Patients with 2 or more risk factors have less than a 50% chance of relapse-free and overall survival at 5 years.

Performance status in the aggressive IPI is defined as follows: Grade Description: 0 Fully active, able to carry on all pre-disease performance without restriction; 1 Restricted in physically strenuous activity but ambulatory and able to carry out work of a light or sedentary nature, e.g., light house work, office work; 2 Ambulatory and capable of all selfcare but unable to carry out any work activities, up to and about more than 50% of waking hours; 3 Capable of only limited selfcare, confined to bed or chair more than 50% of waking hours; 4 Completely disabled, unable to carry on any selfcare, totally confined to bed or chair; and, 5 Dead. (See., The International Non-Hodgkin's Lymphoma Prognostic Factors Project. A predictive model for aggressive non-Hodgkin's lymphoma. N Engl J Med. 329:987-94, 1993)

Typically, the grade of lymphoma is clinically assessed using the criterion that low-grade lymphoma usually presents as a nodal disease and is often indolent or slow-growing. Intermediate- and high-grade disease usually presents as a much more aggressive disease with large extranodal bulky tumors.

The Ann Arbor classification system is also used to measure progression of tumors, especially non-Hodgkins lymphomas. In this system, stages I, II, III, and IV of adult NHL can be classified into A and B categories ' depending on whether the patient has well-defined generalized symptoms (B) or not (A). The B designation is given to patients with the following symptoms: unexplained loss of more than 10% body weight in the 6 months prior to diagnosis, unexplained fever with temperatures above 38° C, and drenching night sweats. Definitions of the stages are as follows: Stage I-involvement of a single lymph node region or localized involvement of a single extralymphatic organ or site. Stage II-involvement of two or more lymph node regions on the same side of the diaphragm or localized involvement of a single associated extralymphatic organ or site and its regional lymph nodes with or without other lymph node regions on the same side of the diaphragm. Stage III-involvement of lymph node regions on both sides of the diaphragm, possibly accompanying localized involvement of an extralymphatic organ or site, involvement of the spleen, or both. Stage IV-disseminated (multifocal) involvement of one or more extralymphatic sites with or without associated lymph node involvement or isolated extralymphatic organ involvement with distant (non-regional) nodal involvement. For further details, see The International Non-Hodgkin's Lymphoma Prognostic Factors Project: A predictive model for aggressive non-Hodgkin's lymphoma, New England J. Med. (1993) 329:987-994.

In one aspect, a therapeutic effect of the CD20-specific binding molecule is determined by the level of response, for example a partial response is defined as tumor reduction to less than one-half of its original size. A complete response is defined as total elimination of disease confirmed by clinical or radiological evaluation. In one embodiment, the individual receiving a single dose of a CD20-specific binding molecule demonstrates at least a partial response to treatment.

According to the Cheson criteria for assessing NHL developed in collaboration with the National Cancer Institute (Cheson et al., J Clin Oncol. 1999, 17:1244: Grillo-Lopez et al., Ann Oncol. 2000, 11:399-408), a complete response is obtained when there is a complete disappearance of all detectable clinical and radiographic evidence of disease and disease-related symptoms, all lymph nodes have returned to normal size, the spleen has regressed in size, and the bone marrow is cleared of lymphoma.

An unconfirmed complete response is obtained when a patient shows complete disappearance of the disease and the spleen regresses in size, but lymph nodes have regressed by more than 75% and the bone marrow is indeterminate. An unconfirmed complete response meets and exceeds the criteria for partial response. An overall response is defined as a reduction of at least 50 percent in overall tumor burden.

Similar criteria have been developed for various other forms of cancers or hyperproliferative diseases and are readily available to a person of skill in the art. See, e.g., Cheson et al., Clin Adv Hematol Oncol. 2006, 4:4-5, which describes criteria for assessing CLL; Cheson et al., J Clin Oncol. 2003, 21:4642-9, which describes criteria for AML; Cheson et al., Blood 2000, 96:3671-4, which describes criteria for myelodysplastic syndromes.

In another aspect, a therapeutic response to a CD20-binding molecule in patients having a B cell cancer is manifest as a slowing of disease progression compared to patients not receiving therapy. Measurement of slowed disease progression or any of the above factors may be carried out using techniques well-known in the art, including bone scan, CT scan, gallium scan, lymphangiogram, MRI, PET scans, ultrasound, and the like.

In a related aspect, to determine the efficacy of CD20-binding molecule treatment the number of B cells in a biological sample of the individual is measured. In one embodiment, the biological sample is selected from blood, tumor biopsy, lymph nodes, tonsils, bone marrow, thymus and other lymphocyte-rich tissue. Lymphocyte-rich tissue is tissue particularly rich in lymphocyte cells, including but not limited to, lymph nodes and related organs (spleen, bone marrow, tonsils, thymus, mucosal lymph tissue), tumors and areas of inflammation.

It will also be apparent that dosing may be modified if traditional therapeutics are administered in combination with therapeutics of the invention.

In one aspect, an individual treated by methods of the invention demonstrates an improved response to treatment with the CD20-binding molecule described herein which is better than the response to treatment with rituximab and no other CD20-binding molecule. An improved response is assessed by comparison of clinical criteria well-known in the art and described herein. Exemplary criteria include, but are not limited to, duration of B cell depletion, reduction in B cell numbers overall, reduction in B cell numbers in a biological sample, reduction in tumor size, reduction in the number of tumors existing and/or appearing after treatment, and improved overall response as assessed by patients themselves and physicians, e.g., using an International Prognostic Index.

It is further contemplated that an individual being treated by a method of the invention may be re-treated, for example, if symptoms of disease reappear or the pharmacokinetics and/or pharmodynamics of the therapeutic make such re-treatment advisable. In one embodiment, the individual treated with a single dose of a CD20-binding molecule is administered another single dose of CD20-specific binding molecule. Based upon ordinary skill in the art, a clinician would be able to identify when re-treatment is indicated based upon, for example, reappearance of disease symptoms or recovery of the individual's B cells to a level requiring re-treatment. Examples of other measurements or markers of clinical criteria and outcome are described further herein. An individual treated by a method of the invention may be placed on a maintenance schedule of treatment, wherein the individual is re-treated with a single dose of CD20-specific binding molecule based on pharmacokinetic/pharmacodynamic properties of the CD20-specific binding molecule. Such a maintenance treatment is typically administered anywhere from about three months to about two years after the initial single dose. Exemplary pharmacodynamic data include, but are not limited to, biological measures for improvement of disease as described herein, such as levels of CD20-specific binding molecule in serum, improvement in disease assessment (e.g., by ACR, SLAM or IPI), change in cytokine or surface marker expression, levels of autoantibodies, and change in tumor size. It is further understood in the art that differences in individual responses to treatment by methods of the invention may necessitate differences in timing of re-treatment with a single dose of CD20-specific binding molecule.

As an additional aspect, the invention includes kits which comprise one or more compounds or compositions packaged in a manner which facilitates their use to practice methods of the invention. In one embodiment, such a kit includes a CD20-specific binding molecule compound or composition described herein (e.g., a composition comprising a CD20-specific binding molecule alone or in combination with a second agent), packaged in a container such as a sealed bottle or vessel, with a label affixed to the container or included in the package that describes use of the compound or composition in practicing the method. Preferably, the compound or composition is packaged in a unit dosage form. The kit may further include a device suitable for administering the composition according to a specific route of administration or for practicing a screening assay. Preferably, the kit contains a label that describes use of the antibody composition.

The present invention also comprises articles of manufacture. Such articles comprise at least one CD20-specific binding molecule, optionally together with a pharmaceutical carrier or diluent, and at least one label describing a method of use of the CD20-specific SMIP according to the invention. Such articles of manufacture may also optionally comprise at least one second agent for administration in connection with the CD20-specific SMIP.

The present invention also calls for use of a composition comprising at least one CD20-specific binding molecule in the manufacture of a medicament for the inhibition or prevention of aberrant B-cell activity, or for the treatment or prophylaxis of a disease, condition, or disorder in a subject characterized or mediated by aberrant B-cell activity.

### BRIEF DESCRIPTION OF THE DRAWINGS

Figure 1A illustrates assessment of B cell depletion in a dose range study of non-human primates receiving a single dose of the CD20-specific binding molecule TRU-015. Figure 1B is a further analysis of B cell depletion in a similar experiment measured over a longer time course and shown as percent B cell depletion.

Figure 2 illustrates assessment of B cell depletion in human patients as a result of single dose administration of the CD20-specific binding molecule TRU-015.

Figure 3 shows a comparison of TRU-015 and other CD20-binding molecule constructs in ADCC and CDC assays.

Figure 4 shows the duration of B cell depletion, as measured by the number of CD19+ B cells, in RA patients receiving TRU-015 in a dose range study.

Figure 5 shows B cell depletion in RA patients receiving a single dose of 15 mg/kg TRU-015 or 2X 7.5 mg/kg dose TRU-015.

### EXAMPLES

Additional aspects and details of the invention will be apparent from the following examples, which are intended to be illustrative rather than limiting. Example 1 describes recombinant production of a CD20-specific SMIP. Example 2 describes a CD20-specific SMIP activity in vitro. Example 3 describes effects of a CD20-specific binding molecule on a B-cell tumor line in vivo. Example 4 describes single -dose administration of a CD20-specific SMIP to non-human primates. Example 5 describes the administration of a CD20-specific SMIP to human patients. Example 6 describes administration of a CD20-specific SMIP to treat idiopathic inflammatory myopathy. Example 7 describes administration of a CD20-specific SMIP to treat patients with rheumatoid arthritis. Example 8 describes clinical results of administration of a CD20-specific SMIP to rheumatoid arthritis patients.

### EXAMPLE 1

### RECOMBINANT PRODUCTION OF CD20-SPECIFIC BINDING MOLECULE

CD20-specific SMIPs are described in co-owned US Patent Publications 2003/133939, 2003/0118592 and 2005/0136049. An exemplary SMIP, TRU-015, was selected for further study as described below.

TRU-015 is a recombinant (murine/human) single chain protein that binds to the CD20 antigen. The binding domain was based on a publicly available human CD20 antibody sequence. The binding domain is connected to the effector domain, the CH2 and CH3 domains of human IgG1, through a modified CSS hinge region. TRU-015 exists as a dimer in solution and the dimer has a theoretical molecular weight of approximately 106,000 daltons.

TRU-015 comprises the 2e12 leader peptide cloning sequence from amino acids 1-23 of SEQ ID NO: 2; the 2H7 murine anti-human CD20 light chain variable region with a lysine to serine (VHL11S) amino acid substitution at residue 11 in the variable region, which is reflected at position 34 in SEQ ID NO: 2; an asp-gly₃-ser-(gly₄ser)₂ linker, beginning at residue 129 in SEQ ID NO: 2; the 2H7 murine anti-human CD20 heavy chain variable region, which lacks a serine residue at the end of the heavy chain region, i.e., changed from VTVSS to VTVS; a human IG1 Fc domain, including a modified hinge region comprising a (CSS) sequence, and wild type CH2 and CH3 domains. The nucleotide and amino acid sequences of TRU-015 are set out in SEQ ID NO: 1 and 2, respectively.

The CHO cells that produce TRU-015 were cultured in a bioreactor using proprietary media. TRU-015 was purified using a series of chromatography and filtration steps including a virus reduction filter. The material was then concentrated and formulated with 20 mM sodium phosphate and 240 mM sucrose, with a resulting pH of 6.0. The composition is filtered before filling into glass vials at a concentration of 10 mg/mL. Each glass vial contains 5 mL of TRU-01 5 (50 mg/vial).

### Dosage Form and Administration

TRU-015 is supplied in single-use, glass vials containing 50 mg TRU-015 (5 mL [10 mg/mL]) in a sterile, preservative-free, liquid formulation containing 20 mM sodium phosphate and 240 mM sucrose, with a pH of 6.0. TRU-015 is administered as an intravenous (IV) infusion. Dosing is done by body weight (mg/kg). Vials of TRU-015 are stored frozen at -20°C until use. Following thawing, vials are used immediately or stored at 2 to 8°C.

### EXAMPLE 2

### CD20-SPECIFIC BINDING MOLECULE ACTIVITY IN VITRO

In order to evaluate the efficacy of the CD20-specific SMIP in vivo, the in vitro effects of administration on cell-specific activity, such as Antibody Dependent Cellular Cytotoxicity (ADCC) Activity, Complement Dependent Cytotoxicity (CDC) activity and apoptotic activity were first measured.

### Antibody Dependent Cellular Cytotoxicity (ADCC) Activity,

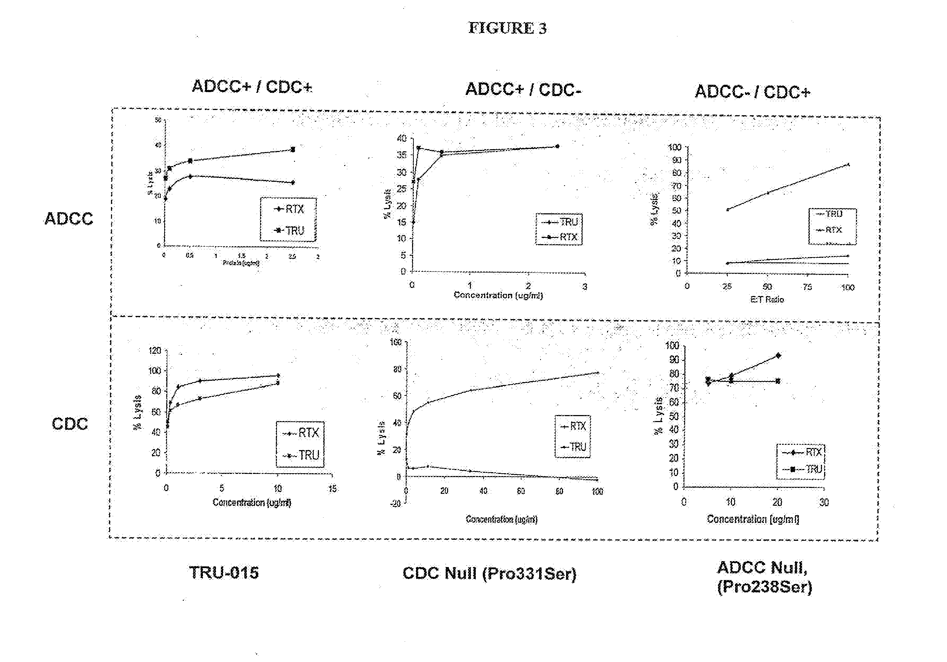

The ADCC activity of TRU-015 has been assessed against a B-cell target (BJAB B lymphoma cell line) using varying doses of TRU-015 or rituximab. The effect of TRU-015 on fresh human peripheral blood mononuclear cells (PBMC, which contain NK cells but no neutrophils) was also measured. Effector cells from 2 different donors demonstrated approximately 30% lysis at both 0.5 and 2.5 µg/ml (Figure 3). In this assay, TRU-01 5 was comparable to rituximab in its ability to induce lysis of CD20+ target cells via ADCC. The CD20-binding molecule similar to TRU-01 5 but having a proline to serine mutation at residue 331 (Pro331Ser) in the effector region demonstrates ADCC activity while the construct having a mutation from proline to serine at residue 238 (Pro238Ser) is null for ADCC activity.

### Complement Dependent Cytotoxicity (CDC) Activity of TRU-015

Complement activation has been reported to be associated with infusion reactions (van der Kolk, Br. J Haematol. 2001, 115:807-11) with rituximab. Additionally, disease activity in chronic inflammatory diseases such as rheumatoid arthritis may be related to complement activation. Therefore, CDC activity in TRU-015 has been attenuated to address these issues. To assess the level of CDC attenuation of TRU-015, CDC activity was assessed and compared to that of rituximab.

Complement dependent cytotoxicity is initiated by the binding of C1q, a constituent of the first component of the complement cascade, to the CH2 domain of TRU-015, when TRU-015 is bound to CD20, the target antigen. To evaluate CDC activity, TRU-015 was incubated with WIL2-S cells in the presence of rabbit complement [Dynal Biotech (Invitrogen), Brown Deer, WI] (Gazzano-Santoro, et al., J Immunol. Meth. 1997, 202:163-71). This experiment may also be carried out using human serum and human complement (C1q) (Quidel Corporation, San Diego, CA).

TRU-015 mediated killing of WIL2-S cells in a dose-dependent manner, showing approximately 30% lysis at 1 µg/ml, 50% lysis at 10 µg/ml and approximately 65% lysis at 1 µg/ml, while a control protein (human IgG) was unable to mediate CDC in these cells. The cytotoxic effect of TRU-015 on WIL2-s cells was complement dependent since TRU-015 in the absence of complement (media only) showed no cytotoxic activity. These results show that recruitment of the complement pathway is another mechanism by which TRU-015 exhibits cytotoxicity. However, compared with rituximab, TRU-015 was 10- to 100-fold less active in its relative depletion of CDC20+ target cells via CDC, indicating that TRU-015 is successfully attenuated in its ability to lyse cells by CDC. Conversely, the CD20-binding molecule similar to TRU-015 but having a proline to serine mutation at residue 331 (P331S) in the effector region demonstrates no CDC activity while the construct having a mutation from proline to serine at residue 238 (P238S) demonstrates CDC activity comparable to the TRU-015 construct (Figure 3).

These assays demonstrate the effector function of the SMIP molecules can be modulated by altering the sequence of the molecule to either improve, maintain, or delete certain effector functions.

### Apoptotic Activity of TRU-015

TRU-015 and rituximab were tested for their ability to induce apoptosis in a CD20+ B-cell Lymphoma line (Ramos B-cells). Induction of apoptosis was quantitated by binding of annexin V/propidium idodide supplied with a commercial assay kit (BD/Pharmingen) by use of a flow cytometry assay following the manufacturer's protocol. TRU-015 and rituximab were similar in their apoptotic activity, demonstrating approximately 60% cell lysis at both 10 and 20 µg/ml.

### Binding Specificity

The specificity of TRU-015 binding to rat splenocytes, mouse splenocytes, monkey peripheral blood, and the CD20 expressing human B-cell lymphoma cell line WIL2-S was assessed with a FACS Caliber Flow Cytometer (BD/Pharmingen). High binding affinity was observed with TRU-015 on human WIL2-S cells and monkey peripheral blood, while no specific binding of TRU-015 was detected in the case of rat or mouse splenocytes. Directly FITC conjugated antibodies to human, mouse, and rat B-cell markers were used as controls for this experiment. Specific B-cell binding was observed in all 3 cell preparations when stained with commercially purchased antibodies to B-cell surface molecules. The results are summarized in Table 1 below and expressed as percent positive staining cells.

**Table 1**

| | **Control** | **CD3** | **CD19** | **GD45R** | **Thru-015** | **Rituximab** |
|---|---|---|---|---|---|---|
| Rat spleen | 9.0 | 52.2 | ND | 30.3 | 5,0 | ND |
| Mouse spleen | 0.14 | 31 | 40.5 | ND | 0,98 | ND |
| Monkey peripheral blood (N=3, mean [SD]) | 0.2 (0.1) | ND | ND | ND | 23.5 (3.9) | 21,5(3.3) |
| Human W1L2-S | 0.43 | ND | 97 | ND | 66 | ND |

These results show that TRU-015 demonstrated significant effector function *in vitro* that is at least equivalent in its ability to induce lysis of CD20+ target cells via ADCC, compared with the CD20-directed monoclonal antibody, rituximab. In contrast, TRU-015 was 10- to 100-fold less active in its relative depletion of CD20+ target cells via CDC, compared with rituximab. In a flow cytometry assay, TRU-015 and rituximab were similar in their apoptotic activity.

### EXAMPLE 3

### EFFECT OF CD20-SPECIFIC BINDING MOLECULE ON A B-CELL TUMOR LINE IN VIVO

Compared with rituximab, Thru-015 demonstrates significant effector function *in vitro* that is at least equivalent in its ability to induce lysis of CD20+ target cells via ADCC. TRU-015 is less potent than rituximab in killing CD20+ target cells via CDC and is similar to rituximab in apoptotic activity. TRU-015 does not bind to mouse or rat CD20; thus, the activity of TRU-015 necessitating the mouse studies to be carried out with human cell lines.

To further establish the activity of TRU-015 against human cells, the ability of TRU-015 to inhibit the growth of established human CD20-expressing tumors has been examined, using the Ramos cell line (a human B-lymphoblastoid cell line derived from a Burkitt's lymphoma).

Fernale athymic nude mice were implanted with human CD20-expressing Ramos tumors. Eight days after implantation, mice were sorted into groups (n = 6-8 per group) with equivalent tumor volumes and were injected intravenously on days 0, 2,4, 6, and 8 with human IgG as a control (100 µg) or with TRU-015 or rituximab (100 µg). Mean group tumor volume was 370 mm³. Tumors were measured 3 times/week and tumor volumes calculated.

Administration of TRU-015 to mice with established Ramos tumor xenografts resulted in a reduction in tumor volumes and improved survival times compared with rituximab and control treated animals. Of the mice receiving TRU-015, 50% achieved complete regression of their tumors, with survival ≥ 90 days. None of the mice in the rituximab group were able to completely resolve their tumors. Median survival time for TRU-015 treated animals was 64.5 days, compared with 11 days and 8 days for rituximab and control treated animals, respectively.

Additional experiments using increased numbers of test animals provided additional statistically significant results. The total numbers of mice used per group was raised to n=40-44 animals and the mean average of survival time and change in tumor size calculated. Survival rates for the increased data sets improved to 90 days for TRU-015 treated animals and approximately 50 days for rituximab treated mice. Mean baseline tumor size was 231 mm³. Analysis of the presence of tumors in these animals showed that approximately 50% of TRU-01 5 mice remained tumor free for 90 days while mice receiving rituximab or control treatment were not tumor free.

Further experiments were carried out by implanting mice with B cell lines which have demonstrated resistance to killing by rituximab. Athymic nude mice were implanted with 5 x 10⁶ Daudi tumor cells which are resistant to killing by rituximab and mice were allowed to develop tumors. Seven days post-implantation mice were divided into groups and given treatment on days 0, 2, 4, 6 and 8 with TRU-015 (100 µg), rituximab (100 µg), control HulgG (100 µg), or PBS. Dose studies were also performed using doses of 30, 300 and 1000 µg/dose. Tumors were measured 3 times/week and tumor volumes calculated,

Administration of TRU-015 to mice with established Daudi tumor xenografts resulted in a reduction in tumor volumes and improved survival times compared with rituximab and control treated animals. Mice receiving TRU-015 at the 1000 µg dose demonstrated a survival rate of approximately 85% 30 days after implantation, decreasing to approximately 65% by day 40. Mice receiving 300 µg TRU-045 per dose showed a survival rate of 85% at day 40, whereas animals receiving 100 µg TRU-015/dose had a survival rate of approximately 65% by day 25, decreasing to 50% at day 40, and showing a median survival time (MST) of approximately 36 days. The MST could not be calculated for the groups receiving 1000 and 300 µg TRU-015 as over 50% of the group members survived longer than the assays were carried out, Mice receiving 30 µg TRU-015 demonstrated approximately 25% survival rate at day 25, showing a MST of 23 days.

Rituximab treated animals demonstrated lower survival rates compared to animals receiving TRU-015. Animals receiving rituximab at 1000 µg/dose had a survival rate of approximately 65% at day 20 decreasing to 25% at day 40, with a MST of 24 days. Mice receiving 300 µg rituximab/dose showed a 50% survival rate at day 20 decreasing to 25% by day 40, with a MST of 24 days. Mice receiving either 100 µg rituximab or 30 µg rituximab/dose both demonstrated a 25% survival rate at day 20 which decreased to approximately 10% survival by day 40, showing a median survival time of 17 and 16 days, respectively.

Thus, the TRU-015 anti-CD20 SMIP is effective at killing B cell tumors that are resistant to killing by the anti-CD20 chimeric antibody rituximab and significantly prolonging the survival of time of TRU-015 treated animals,

To determine a mechanism by which CD20 might be exerting this effect, natural killer (NK) cell depletion experiments were performed on mice receiving Ramos tumor grafts. Mice were implanted with Ramos tumor lines as described above and on days 0, 4, 8 and 12 all mice were given anti-Asialo GM1 IV antibody (WAKO, Dallas, TX) to deplete NK cells. Mice were then given either TRU-015, rituximab or HulgGIV on days 1,3,5, 7 and 9.

Mice given TRU-015 alone demonstrated a 50% survival rate at day 15, decreasing to approximately 30% at day 35 while mice receiving TRU-015 and NK cell depleting antibody demonstrated a survival rate of approximately 40% at day 15, decreasing to approximately 20% by day 25. Animals receiving rituximab alone demonstrated a survival rate of approximately 50% at day 15, decreasing to 10% by day 27, while mice receiving rituximab plus NK cell depleting antibody showed a survival rate of 30% at day 15, decreasing to 10% at day 20.

These results demonstrate that NK cells may play some role in the mechanism of action of both TRU-015 and rituximab, since depletion of NK cells decreases the efficacy of the B cell depleting treatment.

### EXAMPLE 4

### SINGLE -DOSE ADMINISTRATION OF CD20-SPECIFIC BINDING MOLECULE TO NON-HUMAN PRIMATES

The results described above indicate that TRU-015 is effective at depleting B-cells *in vitro.* To determine the efficacy of B-cell depletion *in vivo*, cynomolgus monkeys were infused with CD20 specific SMIP and the pharmacokinetics and pharmacodynamics of the agent measured.

In the initial treatment regimen, cynomolgus monkeys (n=2/group) received a single IV dose of 10 mg/kg of TRU-015 or 2 other preclinical versions of TRU-015. The percentage of B-cells in the peripheral blood was determined utilizing a non-optimized flow cytometry protocol to follow CD20+ and CDC40+ cells. All 6 animals demonstrated significant depletion of peripheral B-cells and substantial recovery by Day 35.

To measure pharmacokinetic (PK) and pharmacodynamics (PD) of TRU-015 *in vivo,* monkeys were injected IV with either a single dose of 10 mg/kg TRU-015, another preclinical CD20-directed candidate, rituximab, or PBS (n = 3/group). Peripheral blood was taken at baseline, then post dose at 1 and 3 days, weekly for 12 weeks, and every 2 weeks for an additional 12 weeks, to be analyzed by flow cytometry. In this study, rapid depletion of B lymphocyte subsets from the peripheral blood was observed with TRU-015 and rituximab. B-cell recovery with TRU-015 was comparable to rituximab as measured by return of CD40+ or CD20+ cells. The returning cells, however, had less CD20 expression. No adverse safety events related to TRU-015 administration occurred during the treatment.

A dose range study was then performed, in which 18 cynomolgus monkeys (n=3/group) were injected IV with a single dose of TRU-015 (0.01, 0.1, 1.0, or 10 mg/kg) or rituximab (10 mg/kg) or PBS. Peripheral blood samples were taken at various time points and analyzed by flow cytometry. CD19 and CD20 were among the markers evaluated to assess response and recovery.

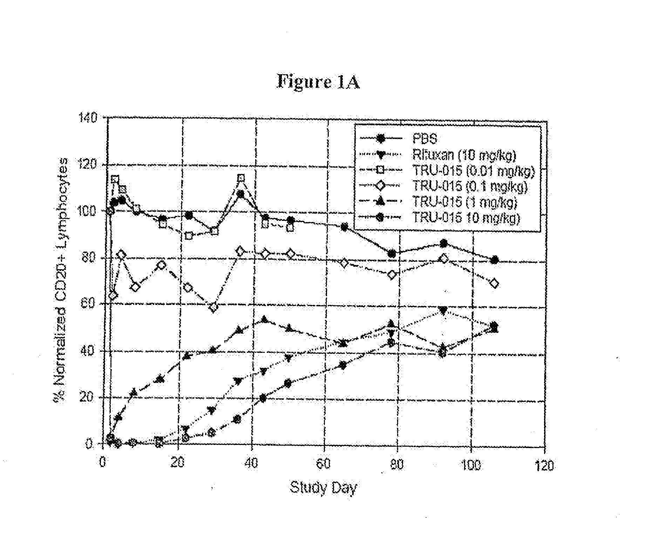

After a single IV administration, the rituximab group and the high dose TRU-015 groups (1.0 and 10 mg/kg) tended to produce similar initial responses in circulating B-cells. In general, B lymphocyte subsets CD19+ and CD20+ in peripheral blood were effectively depleted in all 3 of these treatment groups. The results of the study are shown in Figure 1A.

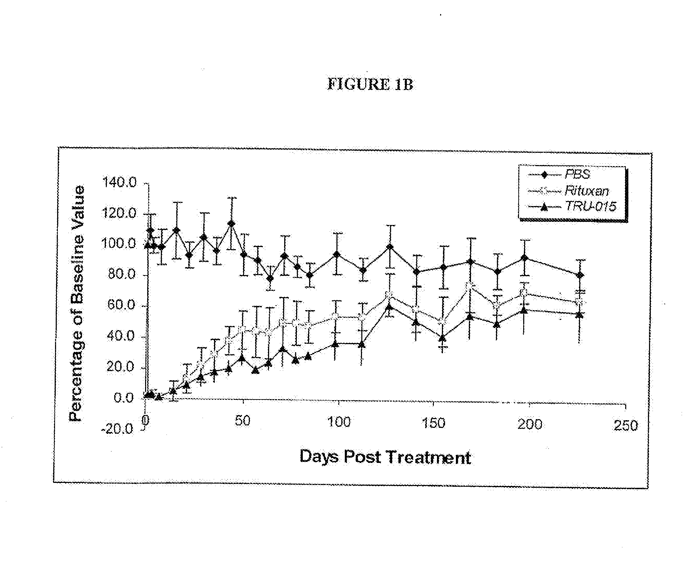

Further analysis of the course of B cell depletion demonstrated that subjects receiving TRU-015 exhibited B cells levels at approximately 30% of baseline levels as far as day 100 post infusion. Depletion was maintained to approximately 55% of baseline levels by day 200, while the rituximab treated groups showed B cell depletion at approximately 60% of baseline at day 200 (Figure 1B).

Results of this dose ranging trial demonstrate that response to TRU-015 is dose dependent. The rituximab group and the 10 mg/kg TRU-015 group showed similar responses in circulating B-cells, with the pattern of B-cell depletion in the 10 mg/kg group replicating that seen in the previous treatment. In general, both were equally effective in depleting B lymphocyte subsets in peripheral blood. Lower doses of TRU-015 demonstrated a dose response, with the 1 mg/kg dose resulting in depletion of B-cells post dose but with a more rapid recovery than the higher dose, the 0.1 mg/kg dose resulting in only partial depletion, and the 0.01 mg/kg dose having no apparent effect.

For PK and PD analysis in the dosed treatments, serum samples from cynomolgus monkeys were analyzed via a flow cytometry-based assay to determine plasma concentration following a single IV injection (slow bolus over 5 minutes) of rituximab at 10 mg/kg or TRU-01 5 at 10, 1, 0.1 million gallon outlining the idea of or 0.01 mg/kg. Serum samples were collected before dosing, at 5 and 30 minutes post dose, at 4, 24, 72, and 168 hours post dose, and on study days 10,14,21, and 28. The plasma concentrations of TRU-015 at doses of 10 mg/kg and 1 mg/kg vs. time were compared with rituximab at 10 mg/kg. TRU-015 and rituximab demonstrated similar plasma concentration levels, with the highest level, TRU-015 at approximately 400 mg/ml at time 0 while rituximab was approximately 450 mg/ml. Plasma concentration of the two compounds decreased similarly to approximately 100 mg/ml at 72 hours post administration. Both rituximab and TRU-015 (10 mg/kg dose) were still detectable in plasma at 168 hours post administration. At the lower dose of TRU-015, the compound was modestly detectable in plasma. Table 2 shows the PK parameters from this treatment regimen.

**Table 2**

| **Dose Group** | | **Cmax (µg/mL)** | **AUC₀₋₂₈ (ug-hr/mL)** | **AUC_{0-Inf} (ug-hr/mL)** | **CL (mL/hr/kg)** | **T½ (hr)** |
|---|---|---|---|---|---|---|
| Rituximab 10mg/kg | Mean | 466 | 28,126 | 28,134 | 0.390 | 57 |
| | SD | 139 | 9,824 | 9,826 | 0.151 | 1 |
| TRU-015 10 mg/kg | Mean | 409 | 27,096 | 27,596 | 0.365 | 114 |
| | SD | 26 | 2,338 | 2,782 | 0,038 | 41 |

| | | | | | | |
|---|---|---|---|---|---|---|
| AUC = area under the concentration-time curve, (ug-hr/mL); Cmax = maximum concentration; SD = standard deviation; T½ = half-life | | | | | | |

Thus, this study demonstrates that the effects of TRU-015 are dose dependent, reversible, and reproducible,

### EXAMPLE 5

### ADMINISTRATION OF TRU-015 TO HUMAN PATIENTS

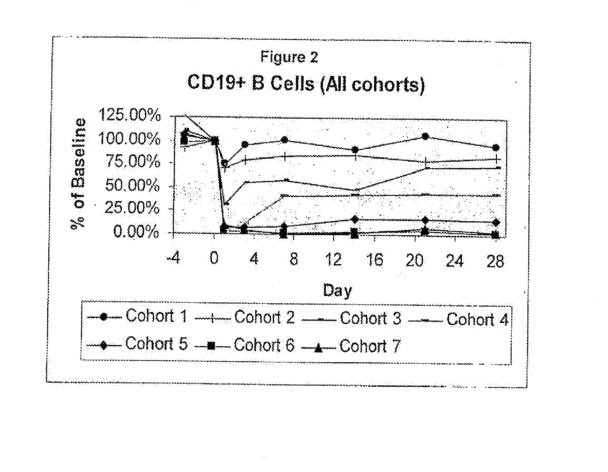

To evaluate the PK and PD of TRU-015 in human subjects, forty individuals with rheumatoid arthritis have been treated with a single dose of TRU-015, ranging from 0.015 to 15 mg/kg. TRU-015 was administered to patients in a single intravenous dose over a time period ranging from 15 minutes to 12 hours. Patients received doses as follows: Cohort 1 - 0.015 mg/kg, Cohort 2 - 0.05 mg/kg, Cohort 3 - 0.15 mg/kg, Cohort 4 - 0.5 mg/kg, Cohort 5 - 1.5 mg/kg, Cohort 6-5 mg/kg, Cohort 7-15 mg/kg and Cohort 8 - 5 mg/kg. Impact on circulating B cells was determined by measuring CD19+ cells in peripheral blood by flow cytometry at various timepoints after TRU-015 infusion. B lymphocyte depletion was demonstrated in these patients in a dose-related manner, expressed by both the degree and duration of B lymphocyte reduction, (Figure 2). Results showed that patients in cohorts 6 and 7 receiving a single dose of 5 mg/kg and 15 mg/kg of TRU-015, respectively, demonstrated almost complete B cell depletion for up to 4 weeks post infusion. Patients receiving 1.5 mg/kg TRU-01 5 showed less than 15% B cell recovery over a 4 week period while patients receiving 0.5 mg/kg exhibited B cells at approximately 45% baseline levels by the end of 4 weeks. No PK data was available from these studies.

These results indicate that TRU-015 administration to human subjects over a range of doses effectively reduces B-cell populations in patients in need of such treatment.

### EXAMPLE 6

### CD20-SPECIFIC SMIP TREATMENT-IN IDIOPATHIC INFLAMMATORY MYOPATHY

Current management of idiopathic inflammatory myopathy (IIM) involves initial therapy with high dose corticosteroids, typically at doses of at least 1 mg/kg/day of prednisone. Methotrexate and azathioprine are often used in combination with corticosteroids for those with either poor prognostic factors or for those resistant to corticosteroids. Intravenous immunoglobulin, cyclophosphamide, cyclosporine, tacrolimus, mycophenolate mofetil, tumor necrosis factor (TNF) antagonists, chlorambucil, and combinations of the above have been used. Most drugs are used without the support of data from controlled trials (Miller, Arthritis and Allied Conditions: A Textbook of Rheumatology. 15th ed. 1614, 2005).

Recently, Levine (Arthritis Rheum. 2005, 52:601-607) published data demonstrating that treatment with the B-cell depleting agent, rituximab, improved the myositis in a cohort of 6 DM patients. Furthermore, anecdotal reports of patients with highly refractory polymyositis have improved with B-cell depleting therapy.

To determine the effects of CD20-specific SMIPs on the progression of IIM, human patients are treated with an exemplary SMIP, TRU-015, and followed for disease course, B-cell depletion, B-cell recovery and other effects of CD20-specific SMIP treatment.

Patients in one treatment group receive either placebo or TRU-015 at a single dose of 5 mg/kg. Patients in a second treatment group are randomized to receive two doses of either placebo or TRU-015 at 5 mg/kg, the first dose administered at Baseline and the second on Day 7. Patients in a third treatment group are randomized to receive either placebo or TRU-01 5 at a single dose of 15 mg/kg. At least 6 subjects with active polymyositis (PM) or dermatomysositis (DM) are enrolled in each treatment group. Each groups incudes approximately 4 active-treated and 2 placebo-treated subjects.

Subjects are evaluated for a minimum of 12 weeks. Subjects with evidence of B-cell depletion are assessed every 4 weeks to monitor disease activity and B-cell recovery. B-cell depletion is defined as a decrease in absolute B-cell count below the lower limit of the normal range. B-cell recovery is defined as a return of absolute B-cell count to either of the following: 1) 70% of subject's baseline value; or 2) normal range.

Before administration of the CD-20-specific SMIP, baseline assessments of disease and other health parameters are obtained. Baseline assessments include measurement of concomitant medication, vital signs (blood pressure, pulse, temperature and respiratory rate), targeted physical examination, manual muscle testing, spirometry, subject global assessment/physician global assessment, disability index by HAQ, Myositis Disease Activity Assessment Tool (MDAAT), flow cytometry, hematology profile (CBC with differential and platelet count), urinalysis, chemistry profile (electrolytes, BUN, creatinine, alkaline phosphatase, total protein, and albumin), LFTs (AST, ALT, LDH), muscle enzymes (CPK, aldolase), quantitative immunoglobulins, blood sample for genotyping, and serum sample for study drug concentration and testing for antibody to TRU-015.

Each subject is pre-medicated with methylprednisolone (e.g., 80LUMEDROL® 100 mg IV), acetaminophen, and an antihistamine (e.g., diphenhydramine, loratadine, or similar product) prior to dosing with TRU-015. or placebo. The physician evaluates each subject and determines the appropriate dose of these pre-medications. The subject then receives the appropriate dose of TRU-015 or placebo by IV infusion.

The subject is assessed in follow-up assessments on Days 1, 7, 14, 28, 47, 56, 70 and 84. Subjects continue to be followed beyond Day 84 at 28-day intervals as deemed clinically appropriate. Subjects are followed at least until B-cell recovery.

Appropriate quantities of TRU-015 are administered to subjects by IV infusion. Dosing of TRU-015 is by body weight (mg/kg). Any subject weighing 110 kg or more, however, is considered to weigh 110 kg for the purposes of dose calculation. TRU-015 is prepared in glass IV bottles with diluent (e.g. 0.9% Sodium Chloride, USP). Infusions of compound are initiated at a rate of 25 mL per hour. After 30 minutes, if no toxicities have been observed, the rate may be increased to 50 mL per hour. The rate may be increased by 25 mL per hour every 20-30 minutes as tolerated (not to exceed 250 mL per hour).

At Day 1 (18 - 24 hours post-dose) patients are assessed for the following procedures: concomitant medications, adverse event assessment, vital signs (blood pressure, pulse, temperature, and respiratory rate), targeted physical examination, flow cytometry, and serum for study drug concentration.

At Day 7 (1 week) patients are assessed for the following procedures: concomitant medication, adverse event assessment, vital signs, targeted physical examination, flow cytometry, hematology profile (CBC with differential and platelet count), urinalysis, chemistry profile (electrolytes, BUN, creatinine, alkaline phosphatase, total protein, and albumin), LFTs (AST, AT, LDH), muscle enzymes (CPK, aldolase), and serum sample for study drug concentration.

At Day 14 (2 weeks), Day 28 (4 weeks), Day 42 (6 weeks), Day 56 (8 weeks), Day 70 (10 weeks). Day 84 (12 weeks), and every 4 weeks thereafter, subjects are assessed for the following procedures: concomitant medication, adverse event assessment, vital signs, targeted physical examination, manual muscle testing, spirometry (Day 84 only), subject global assessment/physician global assessment, disability index by HAQ, Myositis Disease Activity Assessment Tool and quantitative immunoglobulins (Week 24 only).

Each of the following disease activity measures will be evaluated for treatment effect: physician global activity assessment; subject global activity assessment; muscle strength: evaluation of 15 muscle groups (neck flexors, deltoids, biceps, wrist extensors, gluteus maximus, gluteus medius, quadriceps, ankle dorsiflexors), each scored on the Kendall 10-point scale; physical function: Health Assessment Questionnaire (HAQ) disability index; muscle-associated enzymes: CPK, aldolase, AST, ALT, LDH; extra-muscular activity assessment: composite of 6 visual analog scales from the klyositis Disease Activity Assessment Tool evaluating constitutional, cutaneous, gastrointestinal, articular, cardiac, and pulmonary activity.

In addition to the individual outcome measures, subjects are evaluated as to whether they achieve the composite response criteria put forth by the internatfonat Myositis Assessment and Clinical Studies (IMACS) group (Rider, Arthritis Rheum. 2004, 50:2281-90), These preliminary response criteria define a responder as an individual with 20% or greater improvement in at least 3 of the 6 core set measurements (listed above), with no more than 2 of the criteria worse by 25% or more. If muscle strength is worse by 25% or more, the subject cannot be considered a responder.

### Primary Clinical Response Assessments

The primary evaluation for clinical response is improvement of muscle enzyme levels. The primary efficacy analysis is measured as a comparison of the improvement of CPK using an area under the curve (AUC) analysis from baseline through 12 weeks, comparing TRU-01 5-treated subjects with those receiving placebo. Mean CPK and aldolase levels, percent decrease in CPK and aldolase, and time to meaningful improvement (30% decline from baseline) are also evaluated.

A secondary evaluation is based on improvement in muscle strength as determined by manual muscle testing of 15 muscle groups described above, based on improvement of the manual muscle testing score using an AUC analysis from baseline through 12 weeks, comparing TRU-015-treated subjects with those receiving placebo. Additionally, mean muscle strength, percent change in muscle strength, and time to meaningful improvement (12% improvement from baseline) is evaluated, Time to 15% improvement is also evaluated,

Each of the other individual response measures is also evaluated, Mean improvement, percent change, and time to meaningful improvement are assessed using physician and subject global assessments, HAQ disability index, muscle enzymes (other than CPK and aldolase), and extra-muscular activity.

### EXAMPLE 7

### CD20-SPECIFIC SMIP TREATMENT IN PATIENTS WITH RHEUMATOID ARTHRITIS

In patients with RA, B-cells undergo antigen-dependent clonal expansion, affinity maturation, and differentiation into plasma cells, and produce rheumatoid factor, a well-recognized prognostic factor for aggressiveness of RA. Immune complex-mediated inflammation and antigen presentation are additional roles of B-cells that are believed to play a role in the pathogenesis of RA. In studies with human RA synovium-SCID mouse chimera, T cell activation was shown to be B-cell dependent (Takemura et al., J Immunol. 2001, 167:1072-80).

TRU-015 has been adapted for use in inflammatory diseases through attenuation of cell killing by complement dependent cytotoxicity (CDC), Potent ADCC activity, however, has been maintained in TRU-015, and response to TRU-015 may not be as sensitive to CD16 polymorphisms.

To assess the effects of CD20-specific SMIPs on patients with rheumatoid arthritis, two studies are undertaken using an exemplary SMIP, TRU-015.

In a single dose administration, subjects receive a single intravenous (IV) infusion of TRU-015, in a dose escalating fashion, at one of the following levels (≥ 4 subjects per cohort): 0.015, 0.05, 0.15, 0.5, 1.5, 5, 15, and 30 mg/kg. Active RA was not required. The agent is administered intravenously as described above for treatment of IIM, using the same formulation and similar infusion rates.

Preclinical evaluations are performed, collecting the following data: concomitant medication, adverse events as described above, targeted physical examination, vital signs (blood pressure, pulse, temperature and respiratory rate), flow cytometry, hematology profile (CBC with differential and platelet count), coagulation profile (PT/PTT), urinalysis, chemistry profile (electrolytes, BUN, creatinine, AST, ALT, alkaline phosphatase, total protein, and albumin), quantitative immunoglobulins, rheumatoid factor/CCP antibodies, genotyping, serum sample to assess drug concentration and testing for antibody to TRU-016, and serum bank, i.e. banking of serum samples on particular dates for assessment.

At Day 1 (18 - 24 hours post-dose) patients are evaluated for the following concomitant medications, adverse event assessment, targeted physical examination, vital signs, flow cytometry, hematology profile, coagulation profile (PT/PTT), urinalysis, chemistry profile, serum to assess drug concentration

At Day 3 (72 hours) patients are evaluated for the following; concomitant medications, adverse event assessment, targeted physical examination, vital signs, flow cytometry, hematology profile (CBC with differential and platelet count), serum to assess drug concentration.

Day 7 (1 week), Day 14 (2 weeks), Day 21 (3 weeks), Day 28 (4 weeks) Subjects will have the following procedures completed: concomitant medication adverse event assessment, targeted physical examination, vital signs, flow cytometry, hematology profile (CBC with differential and platelet count), coagulation profile (PT/PTT) [Day 7, Day 14 and Day 28], urinalysis [Day 7, Day 14 and Day 28], chemistry profile [Day 7, Day 14 and Day 28], quantitative immunoglobulins [Day 28 only], rheumatoid factor/CCP antibodies [Day 14 and Day 28], serum sample to assess drug concentration [and testing for antibody to TRU-015 on Day 28], Serum bank [Day 28 only]

Subjects with evidence of B-cell depletion will continue assessments as described above at 6 weeks, 8 weeks, 10 weeks, and 12 weeks, until there is evidence of B-cell recovery. Evaluations include the above and additionally, coagulation profile (PT/PTT) [Week 8 and Week 12], urinalysis [Week 8 and Week 12], chemistry profile [Week 8 and Week 12], quantitative immunoglobulins [Week 8 and Week 12], rheumatoid factor/CCP antibodies [weeks 8 and 12 only], serum sample to assess drug concentration [Week 6 and Week 8 only] and testing for antibody to TRU-015 [Week 8 only], and Serum bank [Week 12 only].

*Active disease study:* Patients in the first treatment group are randomized to receive either placebo or TRU-01 5 at single dose of 5 mg/kg. Patients in the second treatment group are randomized to receive two doses of either placebo or TRU-015 at 2.5 mg/kg, the first dose administered at Baseline and the second on Day 7. Patients in the third treatment group are randomized to two doses of either placebo or TRU-015 at 7.5 mg/kg, the first dose administered at Baseline and the second on Day 7. Patients in the fourth treatment group of are randomized to receive either placebo or TRU-015 at a single dose of 15 mg/kg. At least 6 subjects with active PM or DM are enrolled in each treatment group. Each group includes approximately 10 active-treated and 2 placebo-treated subjects.

In addition to the pre-clinical evaluation performed above, patients in this treatment group, having active RA, area assessed for the following: Complete joint assessment (replaced joints should not be evaluated), Subject global assessment/Physician global assessment, Subject assessment of pain, Disability index by Health Assessment Questionnaire (HAQ), Duration of morning stiffness, CRP and ESR.

Each patient is pre-medicated with methylprednisolone (e.g., SOLUMEDROL 100 mg IV), acetaminophen and an antihistamine as described above in Example 3.

Subjects are monitored for a minimum of 4 weeks. Subjects with evidence of B-cell depletion will have assessments every 2 to 4 weeks thereafter to monitor B-cell recovery.

### EXAMPLE 8

### CLINICAL ASSESSMENT OF RHEUMATOID ARTHRITIS PATIENTS AFTER TREATMENT WITH TRU-015

Treatment of RA patients was carried out as described in Example 7 and the effects of TRU-015 treatment were assessed as far out as 42 weeks post-infusion. At least four patients per group received either 0.015, 0.05, 0.15, 0.5, 1.5, 5, 15, or 30 mg/kg (15 mg/kg treatment 2X week) TRU-015. Pharmacokinetic (PK) data were obtained for TRU-015 in these patient groups. Table 3 shows the relative PK data for groups receiving 0.5, 1.5, 5, 15 or 2X15 mg/kg TRU-015.

**Table 3**

| **Group** Mg/kg | **AUC*_{INF}*** (mcg*hr/mL) | **Cmax** (mcg/mL) | Half-life | Half-life |
|---|---|---|---|---|
| | | | hr | CV% |
| 0.5 | 1,342 | 13.8 | 281 | 59.1 |
| 1.5 | 7,082 | 58.2 | 282 | 26.5 |
| 5 | 18,140 | 169 | 315 | 28.6 |
| 15 | 71,753 | 550 | 484 | 42,1 |
| 2x15 | 144,670 | 643 | 352 | 7.5 |

The terminal half-life of TRU-015 ranged from 281-484 hours, showing that TRU-015, even at low doses, has a long half-life *in vivo*, which increases with an increase in dosage. Additionally, no dose-limiting toxicity or serious adverse events were associated with TRU-015 administration. Few adverse side effects of TRU-015 treatment were reported, which included headaches (13.5%) upper respiratory infection (13,5%), bronchitis (10.8%), peripheral edema (13.5%), pruritus (10%), back pain (13.5%), arthralgia (8.1%), cough (8.1%), ecchymosis (8.1%), fatigue (8.1%), nasopharyngitis (8.1%), and urinary tract infection (8.1%). Grade 3 abnormalities were observed in subjects receiving TRU-015, and these included Grade 3 AST/ALT (n=1), Grade 3 low leukocytes (n=2) and Grade 3 low neutrophils (n=3). No Grade 4 and only three Grade 3 adverse events were observed during treatment, including rash/bronchospasm which resolved without ending treatment, arthralgia at two weeks post drug infusion, and hypertension not associated with infusion. One serious adverse event was observed in this experiment, with one TRU-015 patient developing cholecystitis 6-months post-treatment. In a subsequent study, one patient developed prostate cancer and bacteremia/fever post-prostate biopsy 4 weeks post-treatment, and one patient in the placebo group showed exacerbation of previously diagnosed congestive heart failure.

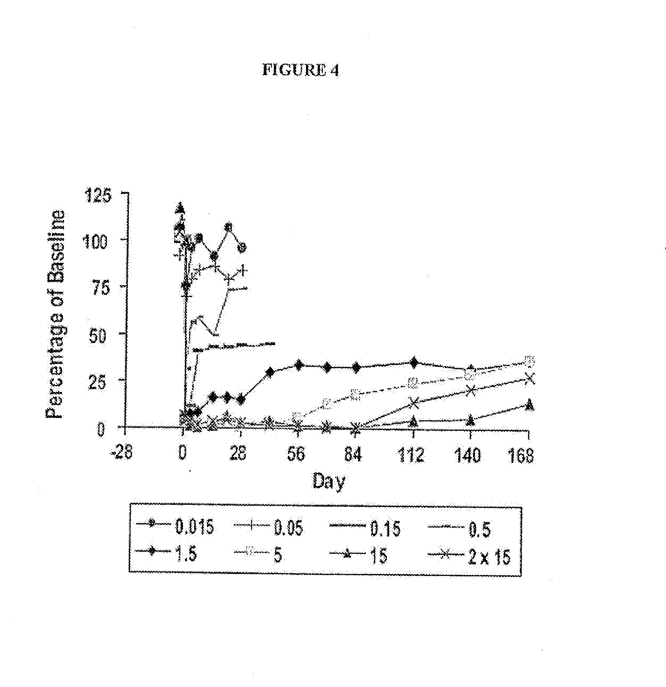

The extent of B cell depletion in RA patients receiving TRU-01 5 was measured and compared to average baseline levels of the patients before treatment began. Figure 4 shows the duration of B cell depletion, as measured by number of CD19+ B cells, in RA patients receiving TRU-015. Patients receiving at least 0.5 mg/kg TRU-015 demonstrated near complete B cell depletion by day one post infusion. Patients receiving either a single dose of 15 mg/kg TRU-015 or 2X 15 mg/kg dose showed B cells completely depleted at day 84 post infusion, rising to approximately 10-15% of baseline by day 168. Patients receiving 5 mg/kg dose showed approximately 15-20% of baseline B cell levels on day 84 post-infusion, increasing to approximately 35% by day 168. Patients receiving a single dose of 1.5 mg/kg TRU-015 demonstrated B cell levels at approximately 30% of baseline at day 84, which remained at that level to day 168.

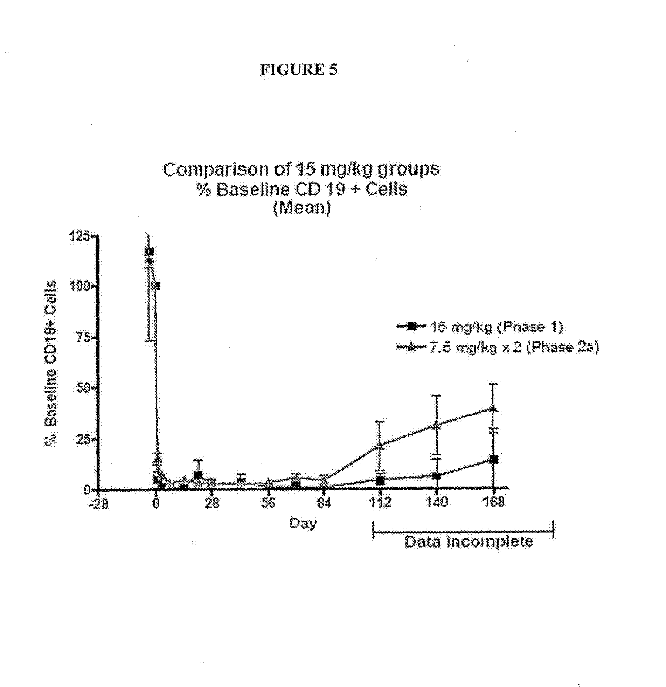

In a second set of treatment regimens, patients were given 5 mg/kg in 2X 2,5 mg/kg doses or given 15 mg/kg in 2X 7.5 mg/kg doses and B cell depletion measured over time. Average B cell depletion in the 2X2.5 mg/kg dose mirrored the B cell depletion of patients receiving 1X 5 mg/kg, dropping to approximately 10% of baseline at day 72 and increasing to approximately 30% of baseline levels by day 172. However, comparison of the patients receiving either 1 dose of 15 mg/kg TRU-015 or 2X 7.5 mg/kg dose showed that at day 84 B cell levels of both groups were almost completely depleted, whereas at day 168, patients receiving the single dose had a slight increase in B cell numbers (to approximately 15%) compared to an increase of up to approximately 35-40% of baseline levels when two doses were administered (Figure 5).

All subjects were evaluated for the presence of neutralizing antibodies to TRU-015. No serum samples tested positive for neutralizing antibodies. Testing of the serum samples from the subjects in the 5 mg/kg and 15 mg/kg group have been evaluated and did not demonstrate neutralizing antibodies. However, TRU-015 was still present in the serum at the time of sampling.

During treatment, patients were also monitored for the levels of Rheumatoid Factor (RF) antibodies and were designated as either RF+ or RF-patients, The maximum clinical response achieved in these groups by 42 weeks after treatment is set out in Table 4.

**Table 4**

| | All subjects | RF+ subjects |
|---|---|---|
| ACR 20 | 76% | 80% |
| ACR 50 | 28% | 35% |
| ACR 70 | 12% | 15% |

These results demonstrate that TRU-01 5 is effective at treating rheumatoid arthritis. TRU-015 therapy provides exposures to a drug that are generally dose proportional and which demonstrate a serum half-life similar to that seen with larger protein therapeutics. A dose response was observed with treatment with TRU-015; with B cell depletion generally increased in degree and duration with increasing dose of TRU-015, showing 100% depletion of cells for an extended period of time. Further, no neutralizing antibodies to TRU-015 have been detected to date.

Numerous modifications and variations in the invention as set forth in the above illustrative examples are expected to occur to those skilled in the art. Consequently only such limitations as appear in the appended claims should be placed on the invention.
The invention will now be defined by the following causes:
1. A method of treating an individual having or suspected of having a disease associated with aberrant B cell activity comprising administering to the individual a therapeutically effective single dose of a CD20-specific binding molecule.
2. A method of treating an individual having or suspected of having a rheumatic disease, comprising administering to the individual a therapeutically effective single dose of CD20-specific binding molecule.
3. The method of clause 2 wherein the rheumatic disease is selected from the group consisting of rheumatoid arthritis, ankylosing spondylitis, dermatomyositis, Henoch Schonlein purpura, juvenile rheumatoid arthritis, psoriatic arthritis, Raynaud's syndrome, Reiter's syndrome, sarcoidosis, spondyloarthropathies, progressive systemic sclerosis and myositis.
4. The method of clause 3 wherein the disease is rheumatoid arthritis.
5. The method of clause 4 wherein the administration of the CD20-specific binding molecule results in an ACR score of 20.
6. The method of clause 4 wherein the number of B cells in a biological sample of the individual is reduced.
7. The method of clause 4 wherein the expression of RANK ligand in a biological sample of the individual is reduced.
8. The method of clause 6 or 7 wherein the biological sample is blood, synovial fluid or synovial biopsy.
9. A method of treating an individual having or suspected of having an inflammatory bowel disease comprising administering to the individual a therapeutically effective single dose of CD20-specific binding molecule.
10. The method of clause 9 wherein the inflammatory bowel disease is selected from the group consisting of ulcerative colitis and Crohn's disease.
11. The method of clause 10 wherein the disease is Crohn's disease,
12. The method of clause 11 wherein administration of the CD20-specific binding molecule results in an improvement in Crohn's Disease Activity Index (CDAI) score in the range of about 50 to about 70 units.
13. The method of clause 10 wherein administration of the CD20-specific binding molecule result in a reduction in perinuclear anti-neutrophil antibody (pANCA) or anti-Saccharomyces cervisiae antibody (ASCA).
14. The method of clause 10 wherein the disease is ulcerative colitis.
15. A method of treating an individual having or suspected of having a central nervous system autoimmune disease, comprising administering to the individual a therapeutically effective single dose of CD20-specific binding molecule.
16. The method of clause 15 wherein the central nervous system autoimmune disease is selected from the group consisting of multiple sclerosis, allergic encephalomyelitis, neuromyelitis optica, lupus myelitis and lupus cerebritis.
17. The method of clause 16 wherein the disease is multiple sclerosis.
18. The method of clause 17 wherein administration of the CD20-specific binding molecule result in a reduction in score on the Expanded Disability Status Scale (EDSS) of at least 0.5.
19. The method of clause 16 wherein the disease is allergic encephalitis.
20. The method of cause 16 wherein the disease is neuromyelitis optica.
21. A method of treating an individual having or suspected of having vasculitis, comprising administering to the individual a therapeutically effective single dose of a CD20-specific binding molecule,
22. The method of clause 21 wherein the vasculitis, is selected from the group consisting of Behcet's disease, central nervous system vasculitis, Churg-Strauss syndrome, cryoglobulinemia, giant cell arteritis, Henoch Schonlein purpura, hypersensitivity vasculitis/angiitis, Kawasaki disease, leucocytoclastic vasculitis, polyantitis, polyarteritis nodosa, polymyalgia, polychondritis, rheumatoid vasculitis, Takayasu's arthritis, Wegener's granulamatosis, vasculitis due to hepatitis, familial Mediterranean fever, microscopic polyangiitis, Cogan's syndrome, Whiskott-Aldrich syndrome and thromboangiitis obliterans.
23. A method of treating an individual having or suspected of having an idiopathic Inflammatory myopathy comprising administering to the individual a therapeutically effective single dose of a CD20-specific binding molecule,
24. The method of clause 23 wherein the inflammatory myopathy is selected from the group consisting of polymyositis and dermatomyositis.
25. The method of clause 24 wherein administration of the CD20-specific binding molecule results in a reduction in at least one of five criteria set out in the Idiopathic Inflammatory Myopathy Criteria (IIMC) assessment.
26. The method of clause 24 wherein administration of the CD20-specific binding molecule results in a reduction in IIM associated factors selected from the group consisting of creatine kinase (CK), lactate dehydrogenase, aldolase, C-reactive protein, aspartate aminotransferase (AST), alanine aminotransferase (ALT), and antinuclear autoantibody (ANA), myositis-specific antibodies (MSA), and antibody to extractable nuclear antigens.
27. The method of clause 24 wherein administration of the CD20-specific binding molecule results in a reduction in creatine kinase (CK) levels.
26. The method of any one of clauses 2, 9, 15, 21 or 23 wherein the binding molecule is administered in conjunction with a second agent.
27. The method of clause 26 wherein the second agent is selected from the group consisting of a second B-cell specific binding molecule, a cytokine, a chemokine, a growth factor, and an immunosuppressive agent.
28. The method of clause 27 wherein the second agent is selected from the group consisting of non-steroidal anti-inflammatory drugs (NSAIDs), analgesiscs, glucocorticoids, disease-modifying antirheumatic drugs (DMARDs) for the treatment of arthritis, and biologic response modifiers.
29. A method of treating an individual having or suspected of having an cancer associated with aberrant B cell activity comprising administering to the individual a therapeutically effective single dose of a CD20-specific binding molecule.
30. The method of clause 29 wherein the cancer is selected from the group consisting of a B cell lymphoma, a B cell leukemia, and a B cell myeloma.
31. The method of clause 29 or 30 wherein the cancer is selected from the group consisting of Hodgkin's disease, non-Hodgkins lymphoma, central nervous system lymphoma, acute lymphoblastic leukemia, chronic lymphocytic leukemia, Hairy cell leukeimia, chronic myoblastic leukemia, small lymphocyte lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extranodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma/leukemia, B-cell proliferations of uncertain malignant potential, lymphomatoid granulomatosis, and post-transplant lymphoproliferative disorder.
32. The method of clause 29 wherein the number of B cells in the individual is reduced.
33. The method of clause 32 wherein a biological sample of the individual selected from the group consisting of blood, tumor biopsy, saliva, lymph nodes, tonsils, bone marrow, thymus and other lymphocyte-rich tissue has a reduced number of B cells.
34. The method of clause 29 wherein the CD20-specific binding molecule is administered in conjunction with a second agent.
35. The method of clause 34 wherein the second agent is selected from the group consisting of a second B-cell specific binding molecule, a cytokine, a chemokine, a growth factor, an immunosuppressive agent, a chemotherapeutic agent and a radiotherapeutic agent.
36. The method of clause 29 wherein the individual demonstrates at least a partial response to treatment with the CD20-specific binding molecule.
37. The method of clause 29 wherein the individual demonstrates a response to treatment with the CD20-binding molecule which is improved in comparison to treatment with rituximab and no other CD20-binding molecule.
38. The method of clause 37 wherein the individual is also administered rituximab.
39. The method of clause 1, 2, 9, 15, 21, 22 or 29 wherein the CD20-specific binding molecule is CD20-specific small, modular immunopharmaceutical (SMIP) TRU-015.
40. The method of clause 39 wherein the CD20-specific SMIP is administered in a dose range of about 0.01 to about 50 mg/kg.
41. The method of clause 40 wherein the CD20-specific SMIP is administered in a dose range of about 0.015 to about 30 mg/kg.
42. The method of clause 41 wherein the CD20-specific SMIP is administered in a dose of about 0.015, about 0.05, about 0.15, about 0.5, about 1.5, about 5.0, about 15 or about 30 mg/kg.
43. The method of clause 39 wherein the CD20-specific binding molecule has an affinity for CD20 in the range of about 1 nM to about 30 nM.
44. The method of clause 39 wherein the CD20-specific binding molecule has a half-life of about 7 to about 30 days in vivo.
45. The method of any one of clauses 1, 2, 9, 15, 21, 22 or 29 wherein the administration of the CD20-specific binding molecule results in reduction in the number of B cells in the individual by at least 20%.
46. The method of clause 45 wherein the number of B cells in the individual is reduced by at least about 20%, about 30%, about 40%, about 50%, about 60%, about 70%, about 80%, about 90% or about 100% as a result of CD20-specific binding molecule administration.
47. An article of manufacture comprising a CD20-specific binding molecule and a label indicating a method according to clause 1.
48. An article of manufacture comprising a CD20-specific binding molecule and a label indicating a method according to clause 2.
49. An article of manufacture comprising a CD20-specific binding molecule and a label indicating a method according to clause 9.
50. An article of manufacture comprising a CD20-specific binding molecule and a label indicating a method according to clause 15.
51. An article of manufacture comprising a CD20-specific binding molecule and a label indicating a method according to clause 21.
52. An article of manufacture comprising a CD20-specific binding molecule and a label indicating a method according to clause 23.
53. An article of manufacture comprising a CD20-specific binding molecule and a label indicating a method according to clause 29.

## Claims

1. A therapeutically effective single dose of a CD20-specific binding molecule for use in a method of treating an individual having or suspected of having a disease associated with aberrant B cell activity.

2. The therapeutically effective single dose of CD20-specific binding molecule for use in a method of treating an individual having or suspected of having a disease associated with aberrant B cell activity of claim 1, wherein the individual has or is suspected of having a rheumatic disease, wherein optionally and preferably the rheumatic disease is selected from the group consisting of rheumatoid arthritis, ankylosing spondylitis, dermatomyositis, Henoch Schonlein purpura, juvenile rheumatoid arthritis, psoriatic arthritis, Raynaud's syndrome, Reiter's syndrome, sarcoidosis, spondyloarthropathies, progressive systemic sclerosis and myositis.

3. The therapeutically effective single dose of CD20-specific binding molecule for use in a method of treating an individual having or suspected of having a disease associated with aberrant B cell activity of any one of the preceding claims, wherein the disease is rheumatoid arthritis and
a) wherein the administration of the CD20-specific binding molecule results in an ACR score of 20; or
b) wherein the number of B cells in a biological sample of the individual is reduced; wherein optionally and preferably, the biological sample is blood, synovial fluid or synovial biopsy or
c) wherein the expression of RANK ligand in a biological sample of the individual is reduced, and wherein optionally and preferably, the biological sample is blood, synovial fluid or synovial biopsy.

4. The therapeutically effective single dose of CD20-specific binding molecule for use in a method of treating an individual having or suspected of having a disease associated with aberrant B cell activity of claim 1, wherein the individual has or is suspected of having an inflammatory bowel disease, wherein optionally and preferably, the inflammatory bowel disease is selected from the group consisting of ulcerative colitis and Crohn's disease.

5. The therapeutically effective single dose of CD20-specific binding molecule for use in a method of treating an individual having or suspected of having a disease associated with aberrant B cell activity of claim 4, wherein the disease is Crohn's disease and:
a) wherein administration of the CD20-specific binding molecule results in an improvement in Crohn's Disease Activity Index (CDAI) score in the range of about 50 to about 70 units; or
b) wherein administration of the CD20-specific binding molecule result in a reduction in perinuclear anti-neutrophil antibody (pANCA) or anti-Saccharomyces cervisiae antibody (ASCA).

6. The therapeutically effective single dose of CD20-specific binding molecule for use in a method of treating an individual having or suspected of having a disease associated with aberrant B cell activity of claim 1, wherein the individual has or suspected of having a central nervous system autoimmune disease, wherein optionally and preferably the central nervous system autoimmune disease is selected from the group consisting of multiple sclerosis, allergic encephalomyelitis, neuromyelitis optica, lupus myelitis and lupus cerebritis.

7. The therapeutically effective single dose of CD20-specific binding molecule for use in a method of treating an individual having or suspected of having a disease associated with aberrant B cell activity of claim 6, wherein the disease is multiple sclerosis and wherein administration of the CD20-specific binding molecule results in a reduction in score on the Expanded Disability Status Scale (EDSS) of at least 0.5.

8. The therapeutically effective single dose of CD20-specific binding molecule for use in a method of treating an individual having or suspected of having a disease associated with aberrant B cell activity of claim 1 wherein the individual has or suspected of having vasculitis, wherein optionally and preferably, the vasculitis is selected from the group consisting of Behcet's disease, central nervous system vasculitis, Churg-Strauss syndrome, cryoglobulinemia, giant cell arteritis, Henoch Schonlein purpura, hypersensitivity vasculitis/angiitis, Kawasaki disease, leucocytoclastic vasculitis, polyantitis, polyarteritis nodosa, polymyalgia, polychondritis, rheumatoid vasculitis, Takayasu's arteritis, Wegener's granulamatosis, vasculitis due to hepatitis, familial Mediterranean fever, microscopic polyangiitis, Cogan's syndrome, Whiskott-Aldrich syndrome and thromboangiitis obliterans.

9. The therapeutically effective single dose of CD20-specific binding molecule for use in a method of treating an individual having or suspected of having a disease associated with aberrant B cell activity of claim 1 wherein the individual has or is suspected of having an idiopathic inflammatory myopathy, wherein optionally and preferably the inflammatory myopathy is selected from the group consisting of polymyositis and dermatomyositis; and wherein optionally and preferably,
a) wherein administration of the CD20-specific binding molecule results in a reduction in at least one of five criteria set out in the Idiopathic Inflammatory Myopathy Criteria (IIMC) assessment; or
b) wherein administration of the CD20-specific binding molecule results in a reduction in IIM associated factors selected from the group consisting of creatine kinase (CK), lactate dehydrogenase, aldolase, C-reactive protein, aspartate aminotransferase (AST), alanine aminotransferase (ALT), and antinuclear autoantibody (ANA), myositis-specific antibodies (MSA), and antibody to extractable nuclear antigens; or
c) wherein administration of the CD20-specific binding molecule results in a reduction in creatine kinase (CK) levels.

10. The therapeutically effective single dose of CD20-specific binding molecule for use in a method of treating an individual having or suspected of having a disease associated with aberrant B cell activity of claim 1, wherein the individual has or is suspected of having systemic lupus erythematosis.

11. The therapeutically effective single dose of CD20-specific binding molecule for use in a method of treating an individual having or suspected of having a disease associated with aberrant B cell activity of any one of the preceding claims wherein the binding molecule is administered in conjunction with a second agent, wherein optionally and preferably:
a) the second agent is selected from the group consisting of a second B-cell specific binding molecule, a cytokine, a chemokine, a growth factor, and an immunosuppressive agent; or
b) the second agent is selected from the group consisting of non-steroidal anti-inflammatory drugs (NSAIDs), analgesiscs, glucocorticoids, disease-modifying antirheumatic drugs (DMARDs) for the treatment of arthritis, and biologic response modifiers.

12. The therapeutically effective single dose of CD20-specific binding molecule for use in a method of treating an individual having or suspected of having a disease associated with aberrant B cell activity of claim 1, wherein the individual has or is suspected of having an cancer associated with aberrant B cell activity, wherein optionally and preferably,
a) the cancer is selected from the group consisting of a B cell lymphoma, a B cell leukemia, and a B cell myeloma; or
b) the cancer is selected from the group consisting of Hodgkin's disease, non-Hodgkins lymphoma, central nervous system lymphoma, acute lymphoblastic leukemia, chronic lymphocytic leukemia, Hairy cell leukemia, chronic myoblastic leukemia, small lymphocytic lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma/leukemia, B-cell proliferations of uncertain malignant potential, lymphomatoid granulomatosis, and post-transplant lymphoproliferative disorder; or
c) wherein the number of B cells in the individual is reduced and wherein optionally and preferably a biological sample of the individual selected from the group consisting of blood, tumor biopsy, saliva, lymph nodes, tonsils, bone marrow, thymus and other lymphocyte-rich tissue has a reduced number of B cells.

13. The therapeutically effective single dose of CD20-specific binding molecule for use in a method of treating an individual having or suspected of having a disease associated with aberrant B cell activity of claim 12 wherein the CD20-specific binding molecule is administered in conjunction with a second agent, wherein optionally and preferably, the second agent is selected from the group consisting of a second B-cell specific binding molecule, a cytokine, a chemokine, a growth factor, an immunosuppressive agent, a chemotherapeutic agent, a radiotherapeutic agent and rituximab, and wherein optionally and preferably, the CD20-specific binding molecule has an affinity for CD20 in the range of about 1 nM to about 30 nM.

14. The therapeutically effective single dose of CD20-specific binding molecule for use in a method of treating an individual having or suspected of having a disease associated with aberrant B cell activity of any one of the preceding claims wherein the CD20-specific binding molecule is CD20-specific fusion protein comprising from amino to carboxy terminus: a human CD20-specific single chain Fv (scFv), a human immunoglobulin hinge region, and a truncated human immunoglobulin constant region, wherein optionally and preferably the CD20-specific fusion protein comprises or consists of the amino acid sequence set forth in SEQ ID No. 2; wherein optionally and preferably, the CD20-specific fusion protein is administered in a dose range of about 0.01 to about 50 mg/kg; or the CD20-specific fusion protein is administered in a dose range of about 0.015 to about 30 mg/kg; or the CD20-specific fusion protein is administered in a dose of about 0.015, about 0.05, about 0.15, about 0.5, about 1.5, about 5.0, about 15 or about 30 mg/kg.

15. The therapeutically effective single dose of CD20-specific binding molecule for use in a method of treating an individual having or suspected of having a disease associated with aberrant B cell activity of any one of the preceding claims wherein the CD20-specific binding molecule is CD20-specific fusion protein comprising from amino to carboxy terminus: a human CD20-specific single chain Fv (scFv), a human immunoglobulin hinge region, and a truncated human immunoglobulin constant region, wherein optionally and preferably the CD20-specific fusion protein comprises or consists of the amino acid sequence set forth in SEQ ID No. 2; wherein optionally and preferably the CD20-specific binding molecule has an affinity for CD20 in the range of about 1 nM to about 30 nM; or wherein the CD20-specific binding molecule has a half-life of about 7 to about 30 days in vivo.

16. The therapeutically effective single dose of CD20-specific binding molecule for use in a method of treating an individual having or suspected of having a disease associated with aberrant B cell activity of any one of the preceding claims, wherein the administration of the CD20-specific binding molecule results in reduction in the number of B cells in the individual by at least 20%; or wherein the number of B cells in the individual is reduced by at least about 20%, about 30%, about 40%, about 50%, about 60%, about 70%, about 80%, about 90% or about 100% as a result of CD20-specific binding molecule administration.

17. The therapeutically effective single dose of CD20-specific binding molecule for use in a method of treating an individual having or suspected of having a disease associated with aberrant B cell activity of any one of the preceding claims wherein the CD20-specific binding molecule is a CD20-specific fusion protein comprising from amino to carboxy terminus: a human CD20-specific single chain Fv (scFv), a human immunoglobulin hinge region, and a truncated human immunoglobulin constant region; wherein optionally and preferably the CD20-specific fusion protein comprises or consists of the amino acid sequence set forth in SEQ ID No. 2; wherein optionally and preferably the CD20-specific fusion protein is administered in a dose of about 1 mg/day, about 5 mg/day, about 10 mg/day, about 20 mg/day, about 50 mg/day, about 75 mg/day, about 100 mg/day, about 150 mg/day, about 200 mg/day, about 250 mg/day, about 400 mg/day, about 500 mg/day, about 800 mg/day, about 1000 mg/day, about 1600 mg/day or about 2000 mg/day.
